Europäisches Patentamt

European Patent Office

Office européen des brevets

(19)

(11) Veröffentlichungsnummer: **0 187 270**

**A1**

(12) **EUROPÄISCHE PATENTANMELDUNG**

(21) Anmeldenummer: 85115517.6

(22) Anmeldetag: 06.12.85

(51) Int. Cl.⁴: **A 41 B 13/02**
**B 32 B 25/08**

(30) Priorität: 12.12.84 CH 5929/84

(43) Veröffentlichungstag der Anmeldung:
16.07.86 Patentblatt 86/29

(84) Benannte Vertragsstaaten:
AT BE CH DE FR GB IT LI LU NL SE

(71) Anmelder: ECOMELT AG
Münsterstrasse 11
CH-6210 Sursee(CH)

(72) Erfinder: Braun, Harald
Spillgässli
CH-6205 Eich(CH)

(72) Erfinder: Lepovsky, Peter
P.O. Box 192
Westborough, MA. 01581(US)

(74) Vertreter: Blum, Rudolf Emil Ernst et al,
c/o E. Blum & Co Patentanwälte Vorderberg 11
CH-8044 Zürich(CH)

(54) **Verfahren zur Befestigung eines elastischen Bandes oder Filmes auf der Oberfläche von bahnförmigen Materialien und Verwendung dieser bahnförmigen Materialien.**

(57) Es wird ein Verfahren zur Befestigung eines elastischen Bandes oder Filmes auf der Oberfläche oder Teilbereichen der Oberfläche von bahnförmigen Materialien beschrieben, mit dem eine Konturführung des elastischen Bandes oder Filmes möglich ist. Es wird durch Coextrusion oder nacheinander erfolgende Extrusion ein Produkt hergestellt, das aus mindestens einer Hotmeltschicht und mindestens einer Schicht eines thermoplastisch verarbeitbaren Elastomermaterials aufgebaut ist. Dieses Produkt wird auf das bahnförmige Material kontinuierlich oder diskontinuierlich so in Form eines Bandes oder Filmes aufgetragen, dass die Hotmeltschicht des Produktes mit dem bahnförmigen Material verklebt wird. Das thermoplastisch verarbeitbare Elastomermaterial kann so ausgewählt werden, dass es für den vorgesehenen Verwendungszweck genau die richtigen Elastikeigenschaften besitzt. Auch das Material der Hotmeltschicht kann so ausgewählt werden, dass mit dem jeweiligen bahnförmigen Material eine optimale Verklebung erreicht wird. Gegebenenfalls kann ein elastisches Band aufgeklebt werden, bei dem sich zwischen der Hotmeltschicht und der Schicht des Elastomermaterials noch eine Schutzschicht oder eine Schicht eines zweiten Elastomermaterials befindet.

Die so erzeugten, bahnförmigen Materialien können dann zu Endprodukten oder Zwischenprodukten geschnitten werden, die eine elastisch geraffte Oberfläche oder elastisch geraffte Oberflächenbereiche aufweisen. Ein typisches Beispiel für ein so herstellbares Endprodukt ist eine Höschenwindel mit elastisch gerafften Beinbereichen und/oder elastisch gerafften Bereichen der Taille.

EP 0 187 270 A1

## TITEL DER ERFINDUNG

Verfahren zur Befestigung eines elastischen Bandes oder Filmes auf der Oberfläche von bahnförmigen Materialien und Verwendung dieser bahnförmigen Materialien.

## HINTERGRUND DER ERFINDUNG

Es sind eine grosse Anzahl an Produkten im Handel erhältlich, die elastisch sind oder elastische Teilbereiche aufweisen. Typische derartige Fertigprodukte sind Wegwerfwindeln, die im Bereich des Beinabschlusses und/oder im Bereich des Bundes oder der Taille mit elastischen Bändern versehen sind, sowie Wäschestücke, wie Leibwäsche, Bett-Tücher und ähnliche Produkte, die elastische Teilbereiche aufweisen.

Bisher bekannte Verfahren zur Herstellung von entsprechenden Fertigprodukten oder Zwischenprodukten, die zur Herstellung entsprechender Fertigprodukte herangezogen werden können, weisen eine Anzahl von Nachteilen auf, insbesondere sind derartige Verfahren oft mit einem hohen Arbeitsaufwand verbunden oder bei ihrer Durchführung tritt ein grosser Materialverschleiss auf.

Ziel der vorliegenden Erfindung war es, ein Verfahren zur Befestigung eines elastischen Bandes oder Filmes auf der Oberfläche oder Teilbereichen der Oberfläche von bahnförmigen Materialien zu entwickeln, wobei dieses Verfahren ohne hohen Arbeitsaufwand und grossem Materialverschleiss durchführbar sein soll.

STAND DER TECHNIK

Es ist seit langem bekannt, dass man Fertigprodukte mit elastischen Teilbereichen herstellen kann, indem man vulkanisierten Gummi oder Gummibänder auf diesen Produkten aufnäht und in sie einwebt. Derartige Arbeitsverfahren benötigen jedoch einen grossen Zeitaufwand.

In der USA Patentschrift Nr. 4 081 301 ist ein Verfahren zur kontinuierlichen Befestigung bestimmter Längen von elastischen Bändern an bestimmten Teilen der Oberfläche eines sich bewegenden, im wesentlichen unelastischen bahnförmigen Materials beschrieben. Bei diesem Verfahren wird das elastische Band gestreckt, auf bestimmte Teile des gestreckten Bandes wird ein Klebemittel aufgetragen und das gestreckte Band mit den mit Klebemittel versehenen Teilen an den gewünschten Stellen des bahnförmigen Materiales verklebt. An denjenigen Stellen, wo das elastische Band nicht mit dem bahnförmigen Material verklebt ist, wird es durchgeschnitten. In Spalte 10, Zeilen 49 - 54 dieser Patentschrift wird erwähnt, dass ein bevorzugt eingesetztes Klebemittel ein Hotmeltklebemittel ist, das nach dem Härten elastomer ist. Als bevorzugtes Beispiel wird in Spalte 11, Zeilen 1 und 2, ein hochviskoses Material auf Aethylen- und Vinylacetatbasis genannt. Das Klebemittel wird mit Hilfe einer Auftragevorrichtung intermittierend und in den

Dr.IM.-vd
25.11.1985

gewünschten Mengen auf das durchlaufende, gestreckte elastische Band aufgetragen.

Ein wesentlicher Nachteil dieses Verfahrens besteht darin, dass diejenigen Teile des Gummibandes, die nicht mit dem bahnförmigen Material verklebt werden, Abfall darstellen, der meist 45 - 50 % des zugeführten Gummibandes beträgt, weil im allgemeinen nur etwa 50 - 60 % des Gummibandes auf das folienförmige Material aufgeklebt und somit zur Plastifizierung des Endproduktes benötigt werden.

Die elastischen Bänder können vorher in verschiedene Längen geschnitten sein und lose aus einem Karton heraus verarbeitet werden. Bei dieser Arbeitsweise tritt in den Kartons oft eine Knotenbildung auf, die zu Produktionsstörungen und zu Materialverlust führt.

In jüngerer Zeit ist jedoch auch die Verarbeitung von Bändern durchgeführt worden, die schmal geschnitten auf eine Spule gewickelt im Handel erhältlich sind. Dabei handelt es sich im allgemeinen um Polyurethanfolien, deren Verarbeitung jedoch leicht zu Problemen führt. Die fraglichen Bänder stellen dünne, schmale Filme dar, die im allgemeinen eine Breite von etwa 6 mm und eine Dicke von 40 µm aufweisen. Bei der Zuführung können derartige Bänder leicht reissen. Ferner besitzen derartige dünne Bänder aus Polyurethanfolien eine mangelhafte thermische Stabilität, sodass bei der Berührung mit dem Hotmelt oder der den Hotmelt abgebenden Düse irreversible plastische Verformungen auftreten können.

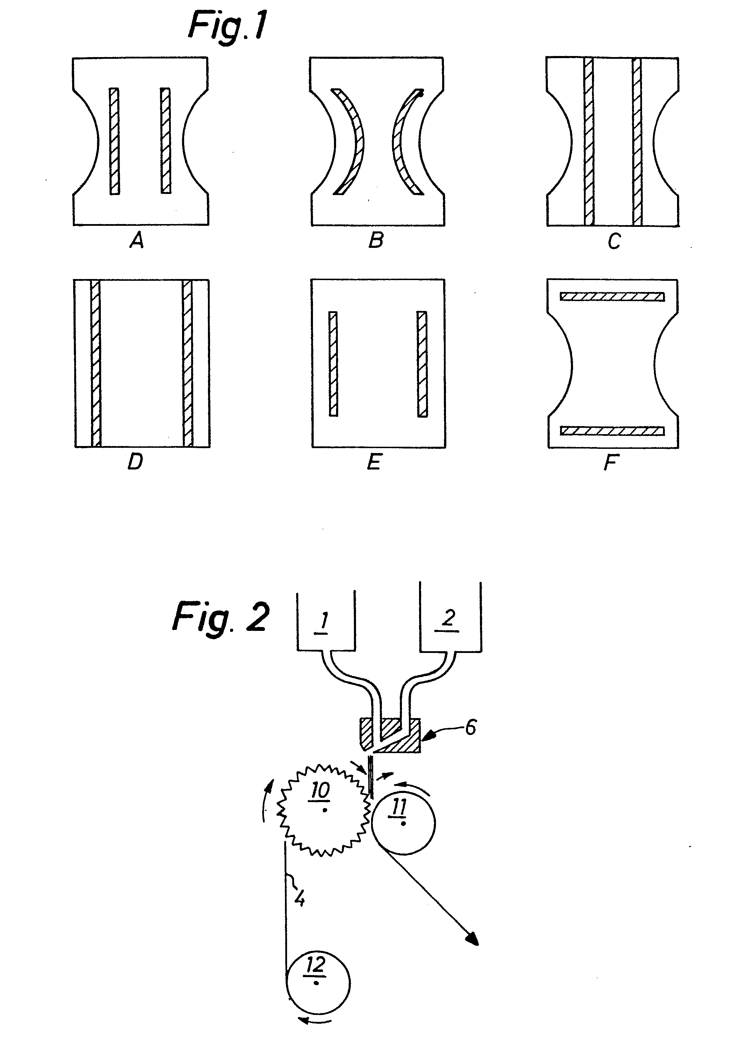

In der europäischen Patentveröffentlichung Nr. 0 115 286 wird ein Verfahren zur Herstellung von Wegwerfwindelhosen beschrieben, die aus einem wasserundurchlässigen Material aufgebaut sind, das plastische Teilbereiche aufweist und die ferner an ihrer Innenseite ein feuchtigkeitsabsorbierendes Kissen tragen. Die elastischen Teilbereiche dieser Windelhosen werden hergestellt, indem man an den entsprechenden Stellen des wasserundurchlässigen folienförmigen Materials eine Mehrzahl von parallel angeordneten elastischen Fäden festklebt. Wie man aus den Figuren 1, 4 und 5 dieser Patentschrift ersehen kann, werden diese parallel verlaufenden elastischen Fäden in gestrecktem Zustand kontinuierlich zugeführt und intermittierend mit einem Hotmeltkleber umhüllt und auf dem folienförmigen Material in gestrecktem Zustand festgeklebt. (Siehe beispielsweise Seite 11, Zeilen 5 - 12 der Beschreibung.) Auch in diesem Falle stellen diejenigen Teile der elastischen Fäden, die bei der diskontinuierlichen Auftragung des Hotmeltklebers nicht von diesem umhüllt wurden und dementsprechend nicht mit dem folienförmigen Material verklebt wurden, Abfall dar. Es sei in diesem Zusammenhang beispielsweise auf die in den Figuren 5 und 6 veranschaulichten Wegwerfwindelhosen verwiesen.

Es ist ferner möglich, den Hotmelt nicht auf das Elastikband diskontinuierlich aufzutragen, sondern auf das bahnförmige Substrat diskontinuierlich aufzutragen und das Elastikband so diskontinuierlich zuzuführen, dass es lediglich auf diejenigen Stellen des bahnförmigen Materiales aufgebracht wird, die mit dem Hotmelt versehen sind. Bei diesem Verfahren ent-

Dr.IM.-vd
25.11.1985

steht zwar kein hoher Abfall an Elastikbändern, aber die diskontinuierliche Zuführung benötigt einen ausserordentlich hohen technischen Aufwand.

Bei dem diskontinuierlichen Verfahren erfolgt nämlich die Zuführung und die Aufklebung des Elastikbandes wie folgt:

Zuführung des verstreckten Elastikbandes auf einem Vakuum/Druckzylinder mit entsprechender Schneidvorrichtung und Uebertragung des Elastikbandes auf das bahnförmige Material. Innerhalb von wenigen Millisekunden muss einerseits die Zuführung des vorher geschnittenen, unter Spannung stehenden Bandes auf den Zylinder erfolgen, der mittels Hochvakuum das unter Spannung stehende Band fixiert und andererseits muss unmittelbar danach das Hochvakuum in einen Ueberdruck umgewandelt werden, um die Uebertragung des Elastikbandes auf das bahnförmige Material zu ermöglichen. Dieses Arbeitsverfahren ist technisch aufwendig, kostenintensiv und bei der Durchführung ist es extrem schwierig, die exakten Bedingungen einzustellen.

Bei allen diesen bisher bekannten Arbeitsverfahren ist ausserdem eine Konturführung des Elastikbandes auf einem bahnförmigen Substrat nicht möglich, und das Elastikband muss in Maschinenlaufrichtung auf das bahnförmige Substrat aufgetragen werden.

Bei dem in der USA Patentschrift Nr. 4 259 220 beschriebenen Verfahren wird versucht, die aufgezeigten Schwierigkeiten zu beheben, indem man einen Hotmelt aus einem Druckkleber aufträgt, wobei dieser Hotmeltkleber sowohl klebende Eigenschaften als auch elastische Eigenschaften besitzt. Der Nachteil dieses elastische Eigenschaften besitzenden Hotmeltklebers ist derjenige, dass er zwei Funktionen gleichzeitig erfüllen muss, und dass dementsprechend Kompromisse bezüglich des klebetechnischen Verhaltens einerseits und des Elastizitätsverhaltens andererseits gemacht werden müssen. So weisen diese, elastische Ei-genschaften besitzenden Hotmeltklebestoffe, ein deut-lich schlechteres Zugdehnungsverhalten im Vergleich zu bisher zu diesem Zweck eingesetzten Elastikbändern auf. Es müssen hochmolekulare Kautschuke, beziehungsweise Kautschuckanteile eingesetzt werden, damit gewährleistet ist, dass der entsprechende Hotmeltklebestoff bei gutem Klebeverhalten eine ausreichende Zugfestigkeit besitzt. Diese hochmolekularen Kautschuke besitzen bei der Verarbeitungstemperatur eine hohe Viskosität, sodass ein Auftragen mit bisher üblichen Hotmeltauftragegeräten nicht möglich ist und spezielle Extruder eingesetzt werden müssen.

Um andererseits ein gutes Klebeverhalten dieser Hotmeltklebestoffe zu gewährleisten, und zwar insbesondere wenn sie auf unpolaren Substraten, wie Polyäthylenfolien, aufgeklebt werden sollen, müssen diese polare, niedrigmolekulare Bestandteile enthalten, wie hauptsächlich aliphatische Harze oder Naturharzderivate. Diese Zusätze jedoch vermindern den Elastizitätsmodel des Hotmeltklebestoffes und auch die Elastizitätsstabilität. Es werden also die Rückstellkräfte im gestreckten Zustand durch einen sogenannten kalten Fluss vermindert, und somit weisen die entsprechenden Produkte nicht die erforderliche Elastizität auf.

Die aufgebrachten Hotmeltkleber zeigen ferner auch nach dem Auskühlen eine gewisse Oberflächenklebrigkeit und sie können somit zu Verklebungen mit anderen Substraten führen, die unerwünscht sind. Dadurch kann auch die vorgesehene Elastifizierung im fertigen Produkt behindert werden.

Ein weiterer schwerwiegenden Nachteil, den die in der USA Patentschrift Nr. 4 259 220 beschriebenen Hotmeltkleber aufweisen, ist ihr schlechtes Rückstellverhalten. Unter Rückstellung versteht man die Kontraktionszeit, die nach dem Einwirken von Dehnkräften zur Rückstellung benötigt wird. Bei üblichen elastischen Materialien, wie Gummi, Polyurethanen und ähnlichen elastischen Produkten, ist eine Rückstellzeit im Bereich von Millisekunden anzutreffen. Bei dem in der genannten USA Patentschrift beschriebenen Hotmeltkleber mit elastomeren Eigenschaften tritt jedoch keine sofortige Rückstellung nach der Einwirkung der dehnenden Kräfte auf, sondern die Rückstellzeit beträgt einige Sekunden. Es ist offensichtlich, dass dies bei den entsprechenden Fertigprodukten zu schlechtem Tragkomfort führt.

BESCHREIBUNG DER ERFINDUNG

Ziel der vorliegenden Erfindung war es, ein Verfahren zur Befestigung eines elastischen Bandes oder Filmes auf der Oberfläche oder Teilbereichen der Oberfläche von bahnförmigen Materialien zu entwickeln, wobei das Verfahren die Nachteile derartiger bisher bekannter Verfahren nicht aufweisen soll. Insbesondere

Dr.IM.-vd
25.11.1985

war man bestrebt, Fertigprodukte oder Zwischenprodukte, die zu entsprechenden Fertigprodukten verarbeitbar sind, herzustellen, bei denen die elastischen Bereiche, bzw. die elastischen Teilbereiche ein gutes Rückstellverhalten aufweisen und keine unerwünschte Klebrigkeit besitzen.

Ueberraschenderweise zeigte es sich, dass die angestrebten Ziele dadurch erreicht werden können, dass man auf das bahnförmige Material mindestens eine Hotmeltschicht und über dieser mindestens eine Schicht eines thermoplastisch verarbeitbaren Elastomermateriales aufbringt.

Ein Gegenstand der vorliegenden Erfindung ist daher ein Verfahren zur Befestigung eines elastischen Bandes oder Filmes auf der Oberfläche oder Teilbereichen der Oberfläche von bahnförmigen Materialien, wobei dieses Verfahren dadurch gekennzeichnet ist, dass man durch Coextrusion oder nacheinander erfolgende Extrusion ein Produkt herstellt, das aus mindestens einer Hotmeltschicht und mindestens einer Schicht eines thermoplastisch verarbeitbaren Elastomermateriales aufgebaut ist und dieses Material auf das bahnförmige Material kontinuierlich oder diskontinuierlich so in Form eines Bandes oder Filmes aufträgt, dass die Hotmeltschicht des Produktes mit dem bahnförmigen Material verklebt wird.

Bei der Durchführung des erfindungsgemässen Verfahrens kann also das thermoplastisch verarbeitbare Elastomermaterial so ausgewählt werden, dass es die für das jeweilige Anwendungsgebiet optimalen Elastikeigenschaften besitzt und auch der Hotmeltkleber kann so ausgewählt werden, dass er auf dem jeweiligen bahnförmigen Material eine optimale Verklebung gewährleistet. Nach dem Erkalten weist das aufgebrachte elastische Band oder der aufgebrachte elastische Film keine Oberflächenklebrigkeit mehr auf. Im Gegensatz dazu mussten die elastischen Bereiche bei den Produkten, die mit dem elastische Eigenschaften aufweisenden Hotmelt hergestellt wurden, oft anschliessend noch mit puderartigen Stoffen, beispielsweise Talkum, behandelt werden, um eine zu starke Oberflächenklebrigkeit zu verhindern.

Gemäss einer bevorzugten Ausführungsart des erfindungsgemässen Verfahrens wird ein Produkt hergestellt, bei dem sich zwischen der Hotmeltschicht und der Schicht des thermoplastisch verarbeitbaren elastischen Materials eine Schutzschicht befindet, die einen direkten Kontakt der Hotmeltschicht mit der Schicht des elastischen Materials verhindert.

Bei dieser Ausführungsart des erfindungsgemässen Verfahrens kann ein Hotmeltmaterial verwendet werden, das mit dem thermoplastisch verarbeitbaren elastischen Material an sich schlecht verträglich wäre, weil durch die dazwischen liegende Schutzschicht ein direkter Kontakt der beiden Komponenten vermieden wird. Ferner kann in diesem Fall auch ein unerwünschtes Auswandern von Bestandteilen des thermoplastisch verarbeitbaren elastischen Materiales in das Material der Hotmeltschicht und umgekehrt bei langer Lagerung der so hergestellten Produkte vermieden werden.

Wenn man ein entsprechendes Produkt herstellen will, bei dem sich zwischen der Hotmeltschicht und der Schicht des thermoplastisch verarbeitbaren Elastomermateriales eine Schutzschicht befindet, die den direkten Kontakt der Hotmeltschicht mit der Schicht des Elastomermateriales verhindert, dann kann man eine entsprechende Coextrusion durchführen, durch die auf das bahnförmige Material die Schicht des Hotmeltklebers, über dieser die Schutzschicht und darüber die Schicht des thermoplastisch verarbeitbaren Elastomermateriales aufgebracht wird oder es kann auf das bahnförmige Material durch eine nacheinander erfolgende Extrusion die Schicht des Hotmeltklebers, über dieser die Schutzschicht und darüber die Schicht des thermoplastisch verarbeitbaren Elastomermateriales aufgebracht werden.

Anhand der Figuren 2, 3 und 4 soll diejenige Ausführungsart des erfindungsgemässen Verfahrens näher erläutert werden, gemäss der durch Coextrusion ein Produkt hergestellt wird, das aus mindestens einer Hotmeltschicht und mindestens einer Schicht eines thermoplastisch verarbeitbaren Materiales aufgebaut ist.

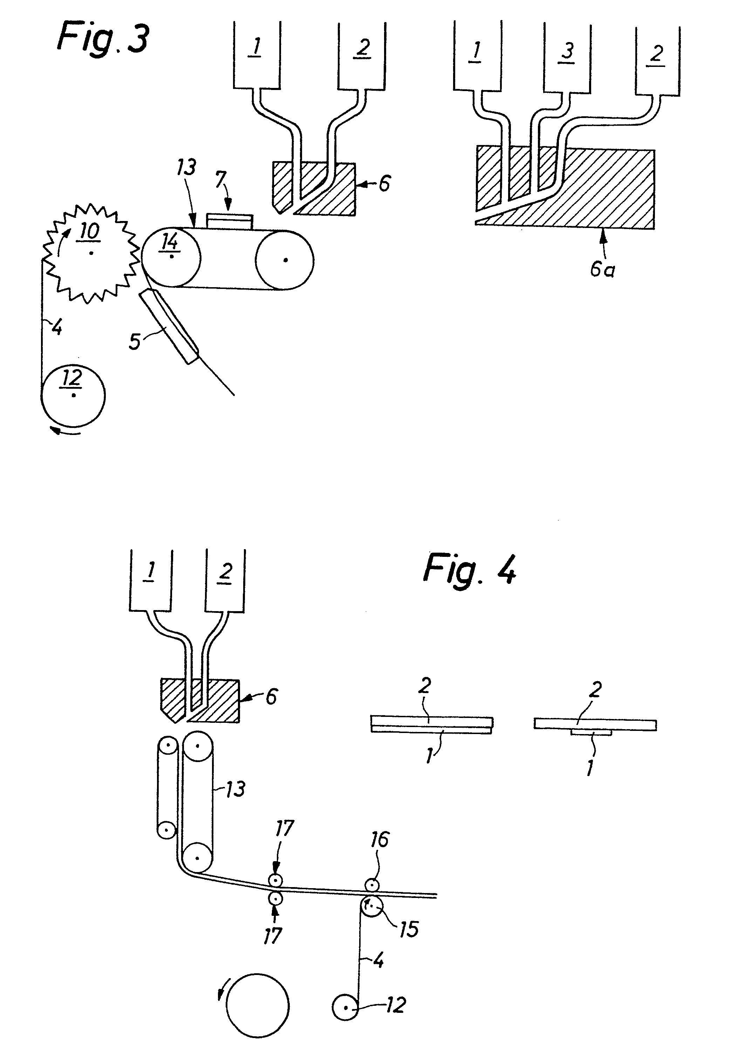

Die zur Durchführung dieses Coextrusionsverfahrens geeignete Extrusionsdüse, die bei den in Fig. 2 und 4 schematisch dargestellten Herstellungsverfahren angewandt wird, weist einen Zuführbehälter (1) auf, der das Material der Hotmeltschicht enthält und einen Zuführbehälter (2), der das Material des thermoplastisch verarbeitbaren Elastomermaterials enthält. Die in Fig. 3 links veranschaulichte Extrusionsdüse zur Durchführung der Coextrusion besitzt

Dr.IM.-vd
25.11.1985

den gleichen Aufbau, d.h. ebenfalls einen Zuführbehälter (1) für das Material der Hotmeltschicht und einen Zuführbehälter (2) für das thermoplastisch verarbeitbare Elastomermaterial.

Bei den Herstellverfahren, die in den Fig. 2, 3 und 4 schematisch dargestellt werden, kann jedoch statt dieser oben beschriebenen Extrusionsdüsen auch eine solche verwendet werden, die zusätzlich zu dem Zuführbehälter (1) für das Material der Hotmeltschicht und dem Zuführbehälter (2) für die thermoplastisch verarbeitbare elastische Komponente noch einen Zuführbehälter (3) aufweist. Diese in Fig. 3 rechts dargestellte Extrusionsdüse ist geeignet, um durch Coextrusion ein Produkt herzustellen, bei dem sich zwischen der Hotmeltschicht (1) und der Schicht des thermoplastisch verarbeitbaren Elastomermaterials (2) noch eine Schutzschicht aus einem Material (3) befindet, die einen direkten Kontakt zwischen der Hotmeltschicht und der Schicht des elastischen Materials verhindert.

Gemäss einer weiteren bevorzugten Ausführungsart des erfindungsgemässen Verfahrens wird durch Coextrusion oder nacheinander erfolgende Extrusion ein Produkt hergestellt, das die Hotmeltschicht und ausserdem zwei oder mehr Schichten des thermoplastisch verarbeitbaren Elastomermateriales aufweist, wobei die unterschiedlichen Schichten des Elastomermateriales unterschiedliche Elastizitäten besitzen. Gegebenenfalls kann ausserdem noch eine Schutzschicht zwischen der Hotmeltschicht und der ihr am nächsten liegenden Schicht des thermoplastisch verarbeitbaren Elastomermateriales vorhanden sein.

Wenn ein entsprechendes Produkt, das zwei Schichten des thermoplastisch verarbeitbaren Materiales aufweist, die unterschiedliche Elastizitäten besitzen, durch eine Coextrusion hergestellt werden soll, dann kann zur Durchführung dieses Verfahrens die in Fig. 3 rechts dargestellte Extrusionsweise herangezogen werden, die drei Zuführbehälter aufweist. Da in diesem Falle das Produkt aus der Hotmeltschicht und zwei Schichten des Elastomermateriales mit unterschiedlicher Elastizität zunächst auf ein gekühltes Förderband extrudiert wird und dann erst mit dem folienförmigen Material verklebt wird, muss in diesem Fall die oberste auf dem Förderband befindliche Schicht die Schicht aus dem Hotmeltmaterial sein. Bei der Herstellung eines derartigen Produktes, wird in dem Zuführbehälter (3) der in Fig. 3 rechts dargestellten Coextrusionsdüse dann das zweite thermoplastisch verarbeitbare elastische Material eingeführt. Wie bereits erwähnt, müssen die beiden thermoplastisch verarbeitbaren Elastomermaterialien unterschiedliche Elastizitäten aufweisen. Im allgemeinen ist es dabei vorteilhaft, wenn die Schicht aus dem zweiten Elastomermaterial eine höhere Elastizität besitzt, als die Schicht aus dem ersten Elastomermaterial. Dadurch gelingt es, ein Endprodukt herzustellen, das in den Bereichen, wo sich nur die Schicht des ersten elastischen Materiales befindet, geringere elastische Eigenschaften aufweist, als in denjenigen Bereichen, wo sich über der Schicht des ersten elastischen Materiales eine weitere Schicht eines zweiten elastischen Materiales befindet.

Es ist offensichtlich, dass die in Fig. 3 rechts dargestellte Düse zur Coextrusion noch so weiter abgewandelt werden kann, dass sie einen vierten Zuführbehälter für Material aufweist, der beispielsweise zur Zuführung eines Materials einer Schutzschicht dient oder zur Zuführung eines Materiales einer dritten Schicht eines thermoplastisch verarbeitbaren Elastomermateriales. Dementsprechend ist es auch möglich, durch Coextrusion Produkte herzustellen, die zwei Schichten eines unterschiedlichen thermoplastischen Materiales aufweisen, sowie eine Schutzschicht, die sich zwischen der Schicht des elastischen Materiales und der Schicht des Hotmeltklebers befindet.

Wenn ein entsprechendes Produkt, das drei oder mehr unterschiedliche Schichten aufweist, durch nacheinander folgende Extrusion der Materialien dieser Schichten hergestellt werden soll, dann kann man die entsprechenden Auftragsvorrichtungen so ausgestalten, dass sich drei oder mehr Extrusionsdüsen hintereinander befinden, durch die jeweils das Material einer Schicht aufgetragen wird, und zwar so, dass die Materialien der einzelnen Schichten dann auf dem Förderband oder dem folienförmigen Material aufeinander zu liegen kommen. Es ist jedoch auch möglich, zwei entsprechende Schichten durch Coextrusion aufzutragen, und darüber dann eine dritte oder eine dritte und vierte Schicht entweder durch nacheinander folgende Extrusion oder Coextrusion der dritten und vierten Schicht aufzubringen.

Dr.IM.-vd
25.11.1985

Das erfindungsgemässe Verfahren kann so durchgeführt werden, dass das coextrudierte Produkt in ungestrecktem Zustand auf das vorgefaltete bahnförmige Material aufgetragen wird. Andererseits ist es auch möglich, das coextrudierte Produkt zunächst abzukühlen, damit es seine elastischen Eigenschaften erlangt, es anschliessend zu strecken und in gestrecktem Zustand auf das nicht vorgefaltete bahnförmige Material aufzukleben. Sobald dann nach dem Aufkleben die Einwirkung der streckenden Kräfte unterbrochen wird, bewirken die Rückstellkräfte der gestreckten Elastikkomponente die Faltung des bahnförmigen Materiales.

In dem zuletzt genannten Fall kann man das coextrudierte Material auf einen Kühlzylinder oder ein Kühlband so extrudieren, dass die Schicht des Elastomermaterials mit dem Kühlband oder Kühlzylinder in Berührung kommt und dass man als Hotmeltschicht eine Selbstklebeschicht verwendet, wobei gegebenenfalls diese Hotmeltschicht diskontinuierlich extrudiert wird, sodass das auf den Kühlzylinder, beziehungsweise das Kühlband coextrudierte Produkt Abschnitte aufweist, die frei von der Hotmeltschicht sind.

Wenn das coextrudierte Produkt nach der weiter oben beschriebenen Arbeitsweise in ungestrecktem Zustand auf das vorgefaltete bahnförmige Material aufgetragen wird, dann kann gemäss einer Verfahrensvariante das coextrudierte Produkt direkt in heissem Zustand mit seiner Hotmeltschicht auf das vorgefaltete bahnförmige Material aufgeklebt werden, und es wird dann während des Aufklebens gekühlt, damit die Elastikkomponente ihre elastischen Eigenschaften noch während

Dr.IM.-vd
25.11.1985

des Aufklebens erhält. Im heissen Zustand besitzt nämlich das Elastomermaterial des beim erfindungsgemässen Verfahren eingesetzten coextrudierten Produktes nicht seine elastischen Eigenschaften. Sollte bei dieser Ausführungsart das Produkt beim Aufkleben nicht gekühlt werden, so würde beim Weiterlaufen des bahnförmigen Materials die Faltung desselben die noch heisse, nicht elastische Schicht des Elastomermateriales strecken, und somit die Faltung und die elastischen Eigenschaften zumindestens teilweise wieder zum Verschwinden bringen.

Gemäss einer anderen vorteilhaften Ausführungsart dieses Verfahrens wird das heisse coextrudierte Produkt nicht direkt auf das vorgefaltete bahn-förmige Material aufgeklebt, sondern zunächst auf eine Uebertragungsbahn oder einen Uebertragungszylinder so aufgebracht, dass die Elastikkomponente mit der Ober-fläche des Uebertragungsbandes oder Uebertragungs-zylinders in Berührung kommt. Dann wird das coextrudierte Produkt auf dem Uebertragungsband oder Uebertragungszylinder so weit abgekühlt, dass das Elastomermaterial seine elastischen Eigenschaften erreicht und anschliessend wird dieses Produkt mit der Hotmeltschicht auf das vorgefaltete bahnförmige Material aufgeklebt. In diesem Falle besteht die Hotmeltschicht vorzugsweise aus einem Selbstklebematerial.

Bei der Durchführung des erfindungsgemässen Verfahrens kann man als bahnförmiges Material beispielsweise ein folienförmiges Kunststoffmaterial, ein vliesförmiges Material, ein nicht gewobenes, gewobenes oder gewirktes Textilmaterial verwenden.

Gemäss einer bevorzugten Ausführungsart des erfindungsgemässen Verfahrens enthält die Schicht des thermoplastisch verarbeitbaren Elastomermateriales des durch Coextrusion oder nacheinander erfolgende Extrusion gewonnenen Produktes

20 - 70 Gew.-%, bezogen auf das Gesamtgewicht der Schicht des Elastomermateriales eines thermoplastischen Kautschukes, der ein Dreiblockcopolymer des Types A-B-A ist, wobei die Komponente A Polystyrolsegmente enthält oder daraus aufgebaut ist und die Komponente B den kautschukartigen Mittelblock darstellt, der vorzugsweise aus Isopren und/oder Butadien-Monomerbestandteilen oder Copoylmerisaten mit anderen äthylenisch ungesättigten Monomeren aufgebaut ist oder ein mindestens teilweise hydriertes Copolymerisat aus mindestens einem Diolefin und mindestens einem Monoolefin ist und

10 - 40 Gew.-%, bezogen auf das Gesamtgewicht der Schicht des Elastomermateriales eines aromatischen Kohlenwasserstoffharzes, das vorzugsweise ein Styrolharz mit Glasübergangstemperaturen und Erweichungspunkten von über 100° C ist.

Vorzugsweise enthält die Schicht des thermoplastisch verarbeitbaren Elastomermateriales noch einen oder mehrere der folgenden Bestandteile:

a) 0-10 Gew.-%, bezogen auf das Gesamtgewicht der Schicht des Elastomermateriales eines Elastomers. Bevorzugte derartige Elastomere sind Butylkautschuke, insbesondere solche mit einer Mooney-Viskosität von ML (1+8 Min.): 41-49 bei 100° C.

b) 0-30 Gew.-%, bezogen auf das Gesamtgewicht der Schicht des Elastomermateriales, eines

Dr.IM.-vd
25.11.1985

aliphatischen Harzes, das eine gute Verträglichkeit mit der Kautschukkomponente B des thermoplastischen Kautschuks aufweist, jedoch eine geringe klebrigmachende Wirkung besitzt. Bevorzugte derartige aliphatische Harze sind Kohlenwasserstoffharze, die einen Erweichungspunkt von $10^{\circ}$ C bis $100^{\circ}$ C, oder höher als $100^{\circ}$ C aufweisen.

c) 0-35 Gew.-%, bezogen auf das Gesamtgewicht des Elastomermateriales eines Weichmachers. Bevorzugte derartige Weichmacher sind aliphatische und/oder naphthenische Oele, Polyisobutylene, Phthalsäureesterprodukte, depolymerisierte Kautschuke oder Mischungen aus zwei oder mehr derartigen Weichmachern.

d) 0-30 Gew.-%, bezogen auf das Gesamtgewicht des Elastomermateriales eines Füllstoffes. Bevorzugt eingesetzte Füllstoffe sind Calciumcarbonat enthaltende oder aus Calciumcarbonat bestehende Füllstoffe.

e) 0,2 - 2 Gew.-%, bezogen auf das Gesamtgewicht des Elastomermateriales mindestens eines Stabilisators.

Als Stabilisator wird vorzugsweise ein Stabilisatorgemisch eingesetzt, das aus mindestens zwei Antioxidantien verschiedener Wirkungsweise aufgebaut ist. Das eine der beiden Antioxidantien ist ein Desinitiator, während das zweite der beiden Antioxidantien ein Kettenabbrecher ist. Als weitere Komponente enthält die Stabilisatormischung im allgemeinen noch ein Lichtschutzmittel.

Der in der bevorzugten Mischung aus Antioxidantien enthaltene Desinitiator schützt die

Dr.IM.-vd
25.11.1985

im erfindungsgemässen Verfahren eingesetzte Schicht des thermoplastisch verarbeitbaren Elastomermateriales, insbesondere vor denjenigen negativen Einflüssen, die bei der Erwärmung dieser Komponente während des Coextrusionsverfahrens oder der nacheinander erfolgenden Extrusion auftreten können. Ferner verhindert jedoch der Desinitiator auch eine Schädigung, die während der Verwendung der Produkte durch den Einfluss von Licht oder Luftsauerstoff auftreten könnte. Die Desinitiatorkomponente verhindert nämlich in erster Linie die Bildung von Hydroperoxiden und/oder sie fördert die Zersetzung von gebildeten Hydroperoxiden. Dadurch kann erreicht werden, dass während der Erhitzung des thermoplastisch verarbeitbaren Elastomermateriales und auch während der Verwendung der Produkte in der Schicht oder den Schichten des Elastomermateriales weniger freie Radikale gebildet werden, die zur Auslösung unerwünschter Kettenreaktionen führen könnten. Als Initiatoren werden bei der Durchführung des erfindungsgemässen Verfahrens vorzugsweise tertiäre Phosphine, Amine und Sulfide, sowie Thioverbindungen eingesetzt.

Der in den bevorzugten Stabilisatormischungen enthaltene Kettenabbrecher führt in Kombination mit dem Desinitiator zu einer gewissen synergistischen Wirkung, d.h. unerwünschte, durch freie Radikale hervorgerufene Reaktionen werden durch den Kettenabbrecher unterbrochen. Als Kettenabbrecher eignen sich insbesonders sterisch gehinderte Phenole.

Als Lichtschutzmittel werden vorzugsweise Ultraviolettlicht absorbierende Produkte eingesetzt. Durch die Absorption des Ultraviolettlichtes
wird verhindert, dass das Ultraviolettlicht die Bildung von freien Radikalen auslöst. Als Ultraviolettlicht absorbierende Mittel werden vorzugsweise Derivate von Benzophenolen und Benzotriazolen eingesetzt.

Die Formulierung des thermoplastisch verarbeitbaren Elastomermateriales oder mehrerer derartiger Elastomermaterialien, die nach dem erfindungsgemässen Verfahren durch Coextrusion oder nacheinander
folgende Extrusion aufgebracht werden, kann so gewählt
werden, dass dem herzustellenden Fertigprodukt genau
diejenigen elastischen Eigenschaften verliehen werden,
die für den vorgesehenen Verwendungszweck optimal geeignet sind. So kann das Zug/Dehnungsverhalten beispielsweise so eingestellt werden, dass im erkalteten
Zustand, beziehungsweise bei der Verwendungstemperatur
des Fertigproduktes, beispielsweise etwa Körpertemperatur, die Elastikkomponente eine Verstreckungsgrenze
bis zu 200 % aufweist. Ferner kann die Kraft pro Querschnitt des Elastomermateriales im erkalteten Zustand
oder bei der Temperatur, bei der das Fertigprodukt verwendet werden soll, 0,2 - 0,8 Newton pro $mm^2$ betragen.

Bei der Durchführung des erfindungsgemässen Verfahrens kann das elastische Band oder der elastische Film auf dem bahnförmigen Material kontinuierlich oder intermittierend befestigt werden. Die Befestigung kann entweder in Maschinenlaufrichtung oder
quer zur Maschinenlaufrichtung oder in Konturform erfolgen. Eine Aufbringung in Konturform auf das bahnförmige Material ist in manchen Fällen speziell geeig-

0187270

net, damit aus diesem bahnförmigen Material Endprodukte hergestellt werden können, die eine optimale Anordnung der elastischen Bereiche gewährleisten. Beispielsweise kann dadurch bei Produkten wie Höschenwindeln ein optimaler Tragkomfort erreicht werden.

Ein weiterer Gegenstand der vorliegenden Erfindung ist die Verwendung des nach dem erfindungsgemässen Verfahren hergestellten bahnförmigen Materials, bei dem auf der Oberfläche oder Teilbereichen der Oberfläche das elastische Band oder der elastische Film befestigt ist, für die Herstellung von Fertigprodukten oder Zwischenprodukten, die eine elastisch geraffte Oberfläche oder elastisch geraffte Oberflächenbereiche aufweisen. Diese Verwendung ist dadurch gekennzeichnet, dass man das bahnförmige Material zu entsprechenden Endprodukten oder Zwischenprodukten zerschneidet.

So kann das nach dem erfindungsgemässen Verfahren hergestellte bahnförmige Material unter anderem zur Herstellung von flüssigkeitsabsorbierenden Produkten, beispielsweise Windeln, Produkten für die Monatshygiene, Produkten für den Krankenhausbereich, wie zum Beispiel Unterlagen für Betten oder Operationstische, Operationsabdecktücher, Kopfbedeckungen, Handschutz und Schuhüberzüge, verwendet werden.

Bei der Herstellung von sogenannten Höschenwindeln ist das mit den elastischen Teilbereichen versehene bahnförmige Material vorzugsweise eine Folie, auf die vor Aufbringung des elastischen Bandes oder Filmes, gleichzeitig mit der Aufbringung des elastischen Bandes oder Filmes oder nach der Aufbringung des ela-

Dr.IM.-vd
25.11.1985

stischen Bandes oder Filmes, die Schicht aus dem flüssigkeitsabsorbierenden Material und vorzugsweise ferner eine Deckschicht aus einem flüssigkeitsdurchlässigen vliesartigen Material ausgebracht wird. Durch Zerschneiden werden dann die entsprechenden fertigen Höschenwindeln erhalten.

In Fig. 1 sind verschiedene Ausführungsarten von nach dem erfindungsgemässen Verfahren hergestellten Höschenwindeln veranschaulicht.

In den Fig. 2, 3 und 4 wird diejenige Variante des erfindungsgemässen Verfahrens, bei der die Auftragung mindestens einer Schicht des thermoplastisch verarbeitbaren Elastomermateriales und der Hotmeltkleber durch Coextrusion erfolgt, näher erläutert.

Bei der in Fig. 2 dargestellten Ausführungsart wird das Coextrudat aus der Hotmeltschicht (1) und der Schicht des elastischen, thermoplastisch verarbeitbaren Materials (2) direkt auf das vorgefaltete, bahnförmige Material (4) extrudiert, wobei nach dem Verkleben sofort mit der Kühlwalze (11) gekühlt wird.

Bei der in Fig. 3 dargestellten Ausführungsart wird das Coextrudat aus der Schicht des thermoplastisch verarbeitbaren Elastomermateriales (2) und des Hotmeltmateriales (1) zunächst auf ein Förderband (13) extrudiert, dort so weit gekühlt, dass das Elastomermaterial seine elastischen Eigenschaften erreicht und dann wird dieses so extrudierte Produkt auf das im vorgefalteten Zustand zugeführte bahnförmige Material (4) aufgeklebt.

Dr.IM.-vd
25.11.1985                    · – 21 –

Bei der in Fig. 4 dargestellten Ausführrungsart erfolgt die Coextrusion des thermoplastisch verarbeitbaren Elastomermateriales (2) und des Hotmeltmateriales (1) ebenfalls auf ein Förderband (13) und dort wird das Produkt gekühlt, bis es seine elastischen Eigenschaften erreicht hat. Anschliessend erfolgt dann eine Verstreckung des coextrudierten Produktes durch Differenzgeschwindigkeit der Walze (17) und der Bahngeschwindigkeit des gekühlten Förderbandes (13). Das gestreckte Produkt wird dann durch den Druck der Walzen 15 und 16 mit dem in nicht gerafftem Zustand zugeführten bahnförmigen Material (4) verklebt.

Die Fig. 2, 3 und 4 werden im Zusammenhang mit Beispielen noch näher erläutert.

In Fig. 1 sind fünf verschiedene Formgebungen der Windel veranschaulicht, wobei die in den Figuren A, B, C, D, E und F schraffierten bandförmigen Bereiche die in gestrecktem Zustand dargestellten elastischen Bereiche des Fertigproduktes veranschaulichen. Wie man sieht, handelt es sich bei den Figuren A, B, C, D und E um elastische Beinabschlüsse der fertigen Windeln, während der elastische Abschnitt in der Fig. F ein elastischer Bundabschluss ist. Selbstverständlich kann ein derartiges Produkt sowohl mit einem elastischen Beinabschluss, beispielsweise dem unter B dargestellten Abschluss, als auch mit einem elastischen Bundabschluss, beispielsweise dem unter F dargestellten Bundabschluss, versehen werden. Ein konturförmig aufgebrachter elastischer Beinabschluss, wie er in Fig. 1B dargestellt wird, führt im allgemeinen zu einem

Dr.IM.-vd
25.11.1985

besonders guten Tragkomfort.

Des weiteren können die nach dem erfindungsgemässen Verfahren hergestellten, mit elastischen Teilbereichen versehen, bahnförmigen Materialien auch zur Herstellung von Kleidungsstücken, Wäschestücken, Bettwäsche und Tischwäsche herangezogen werden, die mit derartigen elastischen Teilbereichen versehen sind. In diesem Fall ist das bahnförmige Material vorzugsweise ein vliesförmiges Material oder ein gewobenes, nicht gewobenes oder gewirktes Textilmaterial.

Des weiteren ist es möglich, die nach dem erfindungsgemässen Verfahren hergestellten, mit elastischen Teilbereichen versehen, bahnförmigen Materialien zu wasserdichten Kleidungsstücken zu verarbeiten, wie zum Beispiel Regenbekleidung, Windjacken und wasserdichte Handschuhen, z.B. Wegwerfhandschuhen aus Kunststoff-Folien.

Wie bereits erwähnt wurde, enthält das thermoplastisch verarbeitbare Elastomermaterial des durch Coextrusion oder nacheinander erfolgende Extrusion erzeugten Produktes, vorzugsweise 20 - 70 Gew.-%, bezogen auf das Gesamtgewicht des Elastomermateriales eines thermoplastischen Kautschuks, der ein Dreiblockcopolymer des Types A-B-A ist.

In diesem Dreiblockcopolymer ist A vorzugsweise ein amorphes Polymer, das eine Glasübergangstemperatur aufweist, die oberhalb der Raumtemperatur liegt. Bei der Verwendung des entsprechenden Fertigproduktes befindet sich daher dieses amorphe Polymermaterial im glasartigen Zustand.

Auch der Mittelblock B des Dreiblockco-

Dr.IM.-vd
25.11.1985

- 24 -

polymeres ist vorzugsweise ein amorphes Polymerisat. Dieses weist jedoch eine Glasübergangstemperatur auf, die weit unterhalb der Raumtemperatur liegt. Bei der Verwendung des entsprechenden Fertigproduktus befindet sich also der Mittelblock B im thermoelastischen Zustand.

Beim Abkühlen einer Schmelze eines derartigen Dreiblockcopolymeres lagern sich die Endblöcke A zusammen und bilden so submikroskopische Teilchen, die als Polymerdomänen bezeichnet werden. Diese Teilchen A, die durch Van-der-Waals'sche-Kräfte zusammengehalten werden, bilden eine diskontinuierliche Phase, während die elastomeren Mittelblöcke B eine kontinuierliche Phase bilden. Jedes einzelne Molekül hat seine Endblöcke A in einer der vielen Polymerdomänen verankert, während sein Mittelblock B sich in der kontinuierlichen Elastomerphase befindet.

Die Polymerdomänen A sind bei Raumtemperatur hart und glasartig. Sie fixieren die Enden der Gummisegmente und wirken als multiple Vernetzungen. Steigt jedoch die Temperatur über die Glasübergangstemperatur der Polymerdomänen A, dann werden die Polymerdomänen flüssig und können unter Belastung zerstört werden, sodass das Polymer unter Druck zu fliessen beginnt. Dementsprechend ist ein derartiges Dreiblockcopolymer des Typs A-B-A nach einer entsprechenden Erhitzung extrudierbar. Sobald das extrudierte Produkt jedoch auf eine Temperatur abgekühlt ist, die sich unterhalb der Glasübergangstemperatur des amorphen Polymeren A befindet, fixieren diese Polymerdomänen A wieder die Enden des elastischen Mittelblocks B, sodass das ursprüngliche Netzwerk wieder hergestellt wird und das Dreiblockcopolymer seine elastischen Eigenschaften wieder erlangt.

Bevorzugte Komponenten B des kautschukartigen Mittelblocks sind, wie bereits erwähnt wurde, Polymerisate oder Copolymerisate aus den Monomeren Isopren und/oder Butadien oder Copolymerisate aus Isopren-Monomeren und/oder Butadien-Monomeren mit anderen äthylenisch ungesättigten Materialien, beispielsweise den Monomeren, Aethylen und Butylen oder mindestens teilweise hydrierte Copolymerisate aus mindestens einem Diolefin und mindestens einem Monoolefin, beispielsweise Copolymerisate, in denen das Monoolefin-Monomermaterial Aethylen oder Butylen oder eine Mischung aus Aethylen und Butylen ist.

Die Komponente A des Dreiblockcopolymeren besteht vorzugsweise aus Polystyrolsegmenten oder enthält Polystyrolsegmente.

Bevorzugte Dreiblockcopolymere des Typs A-B-A, die als thermoplastisch verarbeitbares Elastomermaterial bei der Coextrusion eingesetzt werden, sind daher Styrol-Isoprene-Styrol-Blockcopolymere, die in der Folge als SIS-Blockcopolymere abgekürzt werden, sowie ferner Styrol-Butadien-Styrol-Blockcopolymere, die in der Folge als SBS-Blockcopolymere abgekürzt werden und ferner Styrol-Aethylen-Butylen-Styrol-Blockcopolymere, die in der Folge als SEBS-Blockcopolymere bezeichnet werden.

Von diesen Dreiblockcopolymeren sind wiederum gesättigte SEBS-Blockcopolymere und radial aufgebaute SBS-Copolymere speziell bevorzugt. Durch den Radialaufbau der genannten SBS-Copolymeren, lässt
sich ein hohes Molekulargewicht, d.h. ein hoher Elastizitätsmodul bei niedriger Verarbeitungsviskosität
erreichen. Polymerprodukte mit hydrierten Elastomer-
Mittelblöcken B, wie zum Beispiel die SEBS-Blockcopolymeren, zeichnen sich insbesondere durch eine hohe
Alterungsstabilität aus.

Die beim erfindungsgemässen Verfahren
bevorzugt eingesetzen, oben beschriebenen Dreiblockcopolymeren sind, wie bereits erwähnt wurde, thermoplastisch verarbeitbare Elastomermaterialien, die einen
hohen Elastizitätsmodul bei geringer Verarbeitungsviskosität aufweisen. Diese Eigenschaften unterscheiden die Produkte sehr deutlich, von den in der USA
Patentschrift Nr. 4259220 beschriebenen Elastomeren,
die durch Zusatz von Harzen leicht klebrig gemacht werden können. Die beim erfindungsgemässen Verfahren bevorzugt eingesetzten, oben beschriebenen Dreiblockcopolymeren sind nämlich thermoplastisch verarbeitbare Elastomermaterialien mit schlechtem Klebeverhalten, bzw. kritischer klebrigmachender Wirkung.

Ein weiterer Vorteil, den diese thermoplastisch verarbeitbaren Elastomermaterialien aufweisen, ist derjenige, dass sie im erhärteten Zustand,
d.h. bei Zimmertemperatur, beziehungsweise bei der
Verwendungstemperatur der fraglichen Endprodukte nicht
klebrig sind. Daher ist bei der Verarbeitung die Verwendung von pulverförmigen Materialien, wie zum Beispiel Talcum, zur Unterdrückung einer unerwünschten
Klebrigkeit nicht nötig. Bekanntlich führt die Verwendung von pulverförmigen Materialien, zum Beispiel Talcum, bei Herstellungsverfahren zu unerwünschten Staubentwicklungen, und damit auch zu schlechten arbeitshygienischen Zuständen am Verarbeitungsort.

Bei der Durchführung des erfindungsgemässen Verfahrens muss jedoch als thermoplastisch verarbeitbares Elastomermaterial nicht zwingendermassen ein Dreiblockcopolymer des oben erläuterten Typs A-B-A verwendet werden. Es sind beispielsweise auch Butylkautschuke oder Styrol-Butadien-Kautschuke, die Blockcopolymere sind und in der Folge als SBR-Kautschuke bezeichnet werden, und Naturkautschuke verwendbar. Da diese Produkte jedoch kein ausgeprägtes thermoplastisches Verhalten besitzen, können diese Produkte in der erfindungsgemässen Elastikformulierung nur homogen dispers verteilt werden. Wenn derartige Elastomere als weitere Komponente in dem elastischen thermoplastisch verarbeitbaren Material des durch Coextrusion oder aufeinander folgende Extrusion erzeugte Produktes eingesetzt werden, dann soll ihre Menge zweckmässigerweise höchstens 10 Gew.-%, bezogen auf das Gesamtgewicht der elastisch thermoplastisch verarbeitbaren Komponente, des Elastomers betragen. Höhere Mengen als die angegebenen beeinflussen nämlich das rheologische Verhalten bei der Extrusion sehr nachteilig.

Wie bereits erwähnt wurde, kann das thermoplastisch verarbeitbare Elastomermaterial des extrudierten Produktes als weiteren Bestandteil bis zu 30 Gew.-%, bezogen auf diese elastische Komponente, eines aliphatischen Harzes enthalten, das eine gute Verträglichkeit mit der Kautschukkomponente B des

Dr.IM.-vd
25.11.1985

thermoplastischen Kautschukes aufweist, jedoch eine geringe klebrigmachende Wirkung besitzt. Eine klebrigmachende Wirkung ist bei den erfindungsgemässen Produkten in dem Elastomermaterial unerwünscht. Wenn das Elastomermaterial ein SIS-Polymeres ist, dann soll zweckmässigerweise kein derartiges aliphatisches Harz beigegeben werden, damit eine unerwünschte Klebrigmachung verhindert wird.

Wenn das Elastomermaterial ein SBS-Copolymerisat ist, dann kann gegebenenfalls ein derartiges aliphatisches Harz bis zu der angegebenen Maximalmenge zugesetzt werden.

Aliphatische Harze, wie zum Beispiel hydrierte Kohlenwasserstoffharze aus Monoolefinen mit 5 – 9 Kohlenstoffatomen, die hohe Erweichungspunkte aufweisen, führen jedoch bei Kombination mit SEBS-Copolymerisaten und radial aufgebauten SBS-Copolymeren nur zu schwach klebrigmachenden Eigenschaften oder zu überhaupt keinen klebrigmachenden Eigenschaften. Dementsprechend können diese aliphatischen Harze in diesen Fällen in einer Menge bis zu maximal 30 Gew.-%, bezogen auf das Gesamtgewicht des thermoplastisch verarbeitbaren Elektromateriales eingesetzt werden, um bei der Extrusion des erhitzten elastischen thermoplastischen Materials die Viskosität zu senken.

Wie bereits erwähnt wurde, muss die Schicht des thermoplastisch verarbeitbaren Elastomermateriales zusätzlich zu dem Dreiblockcopolymer des Typs A-B-A noch 10 – 40 Gew.-% eines aromatischen Kohlenwasserstoffharzes enthalten, das vorzugsweise

Dr.IM.-vd
25.11.1985

ein Styrolharz ist, welches einen Erweichungspunkt und eine Glasübergangstemperatur von über 100° C aufweist. Durch diese Komponente wird eine Verstärkung der Endblöcke A des Dreiblockcopolymeren A-B-A erreicht, und damit eine Erhöhung der Hitzebeständigkeit der Produkte. Wichtig ist es jedoch, dass sich diese Styrolharze tatsächlich mit den Endblöcken A des Dreiblockcopolymeren A-B-A assoziieren. Die Polystyrolsegmente des Dreiblockcopolymeren A-B-A besitzen eine Glasübergangstemperatur von etwa 100°C. Eine Erhöhung der Wärmebeständigkeit, bzw. eine Erhöhung der Glasübergangstemperatur dieser Endblöcke A kann nur dann stattfinden, wenn Harze eingesetzt werden, die sich mit den Endblöcken A des Dreiblockcopolymeren A-B-A assoziieren und durch die höhere Glasübergangstemperatur gesamthaft zu einer höheren Glasübergangstemperatur dieser Endblockdomänen beitragen. Aus diesem Grunde werden vorzugsweise aromatische Harze auf Basis von α-Methylstyrol, Vinyltoluol, Copolymerisaten von Cumaron und Inden und Aehnliches verwendet, die alle Glasübergangstemperaturen von über 100°C besitzen.

Als weiteren Bestandteil kann die Schicht des thermoplastisch verarbeitbaren Elastomermateriales des beim erfindungsgemässen Verfahren durch Coextrusion oder nacheinander erfolgende Extrusion hergestellten Produktes, wie bereits erwähnt wurde, bis zu 35 Gew.-%, bezogen auf das Gesamtgewicht der Schicht des Elastomermateriales eines Weichmachers enthalten. Der Weichmacher wird hauptsächlich deshalb beigegeben, um bei der Coextrusion die Viskosität des verflüssigten elastischen Materiales zu senken. Bevorzugt eingesetzte Weichmacher sind aliphatische oder naphtenische Oele oder Mischungen daraus, Polyisobutylene, vorzugsweise solche mit niedrigem Molekulargewicht, depolymerisierte Kautschuke und Phthalsäureesterprodukte. Von diesen Weichmachern sind aliphatische Oele besonders bevorzugt, weil bei ihrer Verwendung eine möglichst starke Erniedrigung der Viskosität bei möglichst geringem Verlust des Elastizitätsmoduls erreicht werden kann. Ferner weisen sie eine gute Mittelblockverträglichkeit auf.

Wie bereits weiter vorne erwähnt wurde, führen auch die gegebenenfalls als weitere Komponente anwesenden aliphatischen Harze zu einer Viskositätssenkung des Elastomermateriales bei ihrer Erhitzung im Zuge des Extrusionsverfahrens. Falls man diese mit SEBS-Copolymerisaten und radial aufgebauten SBS-Copolymeren kombiniert, ist im allgemeinen eine so starke Viskositätssenkung bereits erreicht, sodass die Zugabe des Weichmachers auf Basis eines aliphatischen Oeles sehr stark gesenkt werden kann oder überhaupt kein derartiger Weichmacher mehr zugegeben werden muss.

Im Gegensatz zu den aliphatischen Oelen und naphthenischen Oelen sind Oele mit stärker aromatischem Charakter als Weichmacher nicht geeignet, weil sie die Cohäsion der Domänen der Polystyrolendblöcke erniedrigen.

Zu grosse Mengen an der Weichmacherkomponente führen jedoch auch bei den hier genannten bevorzugten Weichmachern zu einer Verminderung der Cohäsion, sodass die weiter vorne angegebene Maximal-

Dr.IM.-vd
25.11.1985                        - 30 -

menge an 35 Gew.-% Weichmacher, bezogen auf das Gesamtgewicht der Schicht des thermoplastisch verarbeitbaren Elastomermateriales, nicht überschritten werden soll. Eine Ueberdosierung an dem Weichmacher kann gegebenenfalls ausserdem noch zu Migrationsproblemen, also einer Auswanderung der Weichmacherkomponente führen.

Wie bereits weiter vorne erwähnt wurde, kann in der Schicht des thermoplastisch verarbeitbaren Elastomermateriales als weiterer Bestandteil noch ein Füllstoff enthalten sein, und zwar in einer Menge von bis zu maximal 30 Gew.-%, bezogen auf das Gesamtgewicht der Schicht des Elastomermateriales. Obwohl als Füllstoff im allgemeinen Calciumcarbonat bevorzugt ist, können auch andere Füllstoffe eingesetzt werden, wie zum Beispiel Calciumsulfat, Bariumsulfat, Silikate, Kieselsäure und Aluminiumhydroxid. Vorteilhafterweise soll der Füllstoff eine Teilchengrösse bis zu maximal 40 µm aufweisen.

Für die Beurteilung, ob ein Material als Schicht des thermoplastisch verarbeitbaren Elastomermateriales bei der Durchführung des erfindungsgemässen Verfahrens eingesetzt werden kann, sind insbesondere drei Eigenschaften zu beurteilen, und zwar:

I. das Kraft/Dehnungsverhalten,
II. die Retardation und
III. die Rückstellzeit.

Diese drei Eigenschaften des thermoplastisch verarbeitbaren, elastischen Materiales wurden in den nachfolgenden Beispielen nach den folgenden Testverfahren bestimmt:

Dr.IM.-vd
25.11.1985

## I. Prüfung des Kraft/Dehnungsverhaltens

Zur Untersuchung dieser Kenngrössen werden Filme beliebiger Dicke und Breite hergestellt und in der Zugprüfmaschine verstreckt. Die Kraft (F) wird als Funktion der Dehnung ($\epsilon$) gemessen und dies kann durch die folgende Gleichung ausgedrückt werden:

$$F = f \ (\epsilon).$$

Der Querschnitt q der Filme kann aus der Breite der Filme und der Dicke der Filme errechnet werden. Aus diesem errechneten Querschnitt und der gemessenen Kraft F kann der absolute Wert der Kraft $\sigma$ nach der folgenden Formel

$$\sigma = \frac{F}{q}$$

errechnet werden.

Der absolute Wert $\sigma$ der Kraft kann also ebenfalls als Funktion der Dehnung angegeben werden, und zwar gemäss der folgenden Formel:

$$\sigma = f \ (\epsilon).$$

Der gemessene Wert der Kraft (F) ist ferner von der Prüfgeschwindigkeit (V) und der Temperatur (T) abhängig.

Durch mehrmalige Bestimmung der Kraft $\sigma$ mit ein und demselben Prüfkörper entstehen Hysteresekurven, die einen etwaigen Kraftverlust bei Mehrfachdehnung durch plastische Verformung anzeigen. Bei der Bestimmung der Funktion $\sigma = f \ (\epsilon)$ wird ebenfalls die Verstreckungsgrenze (Vg) ermittelt. Oberhalb dieses Grenzwertes findet eine Deformation durch plastische Verformung statt. Diese Verstreckungsgrenze gibt auch den maximalen Dehnwert an, bei dem die Elastikkomponente

0187270

- 33 -

angewendet werden kann.

## II. Bestimmung der Retardation

Die Retardation beschreibt bei einer konstant vorgegebenen Dehnung den Kraftverlust aufgrund plastischer irreversibler Verformungen. Dieser Kraftverlust ist abhängig von der Zeit t und der Temperatur T.

Die Retardation kann nach zwei unterschiedlichen Methoden bestimmt werden:

Bei der ersten Methode wird der Prüfkörper in die Zugprüfmaschine unter Einhaltung eines vorgegebenen Dehnungswertes, beispielsweise 100 %, eingespannt. Es wird dann die Kraft (F) als Funktion der Zeit (t) bei Einhaltung einer konstanten Temperatur bestimmt. Wenn die Temperatur konstant ist und die Dehnung ($\varepsilon$) konstant ist, ist nämlich die Kraft (F) eine Funktion der Zeit, d.h. es gilt die Gleichung

$$F = f(t).$$

Wie aus der Gleichung ersichtlich ist, die im Zusammenhang mit der Bestimmung des Kraft/Dehnungsverhaltens angegeben wurde, gilt dann auch, dass der Absolutwert der Kraft ($\sigma$) bei konstanter Temperatur und bei konstanter Dehnung ($\varepsilon$) eine Funktion der Zeit ist, und zwar gemäss der Formel

$$\sigma = f(t).$$

Gemäss dem anderen Testverfahren zur Bestimmung der Retardation wird ein Prüfkörper der Ausgangslänge X auf eine Länge y · x gedehnt und nach bestimmten Zeitintervallen entspannt. Nach dem Ent-

Dr.IM.-vd
25.11.1985

spannen wird die Länge z des Prüfkörpers nochmals gemessen. Der Unterschied zwischen der Länge z und der Länge x, also der Wert z - x gibt die irreversible plastische Verformung an. Daraus kann der Verformungsgrad (VF) nach der folgenden Formel errechnet werden:

$$VF = \frac{z-x}{x} \cdot 100\,\%.$$

Dieser Verformungsgrad (VF) ist abhängig vom Verstreckungsgrad (y·x), von der Zeit (t) und der Temperatur (T).

### III. Bestimmung der Rückstellzeit

Die Rückstellzeit (tr) ist diejenige Zeit, die ein Prüfkörper der Ausgangslänge X, der auf eine Länge y-x verstreckt wurde, benötigt, um nach dem Entspannen wieder die Ausgangslänge X zu erreichen. Diese Rückstellzeit (tr) ist abhängig vom Verstreckungsfaktor y, der Rückstellkraft F und der Temperatur T.

Für die Beurteilung der Hotmeltschicht sind zwei Kenngrössen von besonderer Bedeutung, nämlich IV die Haftfestigkeit und V die Kriechbeständigkeit.

### IV Bestimmung der Haftfestigkeit der Hotmeltschicht

Es ist wesentlich, dass die Hotmeltschicht eine ausreichende Haftfestigkeit, sowohl gegenüber dem thermoplastisch verarbeitbaren Elastomermaterial als auch gegenüber dem bahnförmigen Material aufweist. Der hier durchgeführte Test zeigt, ob das auf dem bahnförmigen Material nach dem erfindungsgemässen Verfahren befestigte elastische Band eine ausreichende Haftfestigkeit aufweist.

Bei diesem Test wurde eine Coextrusion des thermoplastisch verarbeitbaren Elastomermateriales mit der Hotmeltschicht durchgeführt, und es wurden dabei verschiedene Auftragungsgewichte G der Hotmeltschicht, ausgedrückt in g Hotmeltschicht pro $m^2$ getestet. Die Hotmeltschicht wurde mit dem bahnförmigen Material verklebt, vorzugsweise einem unpolaren Material, indem man das coextrudierte Produkt dazu kaschierte.

Dann wurde in einer Zugprüfmaschine die Abschälfestigkeit beim Abschälen in einem Winkel von 180° bei einer konstanten Abzugsgeschwindigkeit geprüft.

V. Bestimmung der Kriechbeständigkeit

Die Kriechbeständigkeit beschreibt den Widerstand des Films aus dem Hotmeltkleber gegen die Rückstellneigung des Elastomermaterials. Zur Bestimmung dieser Grösse wird aus einem fertigen, nach dem erfindungsgemässen Verfahren hergestellten Produkt ein Teilbereich ausgeschnitten, in welchem das elastische Band mit dem bahnförmigen Material verklebt ist. Dieser herausgeschnittene Bereich hat eine Ausgangslänge X. Er wird auf den vorgegebenen Verstreckungsgrad y·x gedehnt und so fixiert, dass die Elastikkomponente frei auf dem bahnförmigen Material zu liegen kommt und nur von der Hotmeltschicht am Zurückgehen in die Ursprungslage gehindert wird.

Die Kriechbeständigkeit wird bei verschiedenen Temperaturen bestimmt und jeweils wird der Zeitpunkt des Kriechens notiert. Die Kriechbeständigkeit ist ausser von der Temperatur auch noch von der inneren Festigkeit des Klebefilmes, der Haftfestigkeit des Klebefilmes auf der Schicht des Elastomermateriales, sowie von der Rückstellkraft der Schicht des Elastomermateriales abhängig.

Die nachfolgenden Beispiele dienen lediglich zu einer Erläuterung der vorliegenden Erfindung, sie sollen jedoch in keiner Weise als Einschränkung des Erfindungsgedankens ausgelegt werden.

## Beispiel 1

### Herstellung eines thermoplastisch verarbeitbaren Elastomermateriales

Zur Herstellung des Elastomermateriales wurden die folgenden Bestandteile in den in der nachfolgenden Tabelle angegebenen Mengenverhältnissen verwendet:

## Tabelle I

| Bestandteil | Menge in Gew.% |
|---|---|
| Styrol/Äthylen-Butylen-Styrol-Copolymer (28% Styrol / 78% Äthylen-Butylen) | 35 |
| Cycloaliphatisches Harz (Erweichungspunkt ASTM 2398: 100°C) | 15 |
| d-Methylstyrolharz (Erweichungspunkt 120°C) | 15 |
| aliphatisch/naphtenisches Oel ($C_A$ = "%   $C_N$ = 32 %   $C_P$ = 60 %) | 34 |
| Pentaerythrityl tetrakis (3,5-di-tert. butyl-hydroxy-hydrocinnamat) | 0,1 |
| Dilauryl-thio-dipropionat | 0,1 |
| Triphenylphospit | 0,3 |
| Zinkdibutyldithiocarbamat | 0,4 |
| 2-(2'-Hydroxy - 5'methylphenyl) benzotriazol | 0,1 |

Das in der Tabelle genannte SEBS-Copolymerisat weist also ein Styrol/Kautschukverhältnis von 28/72 auf, und es ist das Produkt, das unter der Markenbezeichnung "Kraton G 1650" im Handel erhältlich ist.

Das hohe Molekulargewicht dieses Produktes führt zu folgenden physikalischen Eigenschaften:

Zugfestigkeit:          35 MN/mm2 Bruchdehnung:          500 %.

Das in der Tabelle I genannte cycloali-

Dr.IM.-vd
25.11.1985          - 37

phatische Harz war ein hydriertes Kohlenwasserstoffharz aus Kohlenwasserstoffeinheiten mit 5 bis 9 Kohlenstoffatomen. Sein Erweichungspunkt, bestimmt nach
der Methode ASTM 2398 betrug 100° C und das Molekulargewicht des Produktes 700. Das Harz ist unter der Markenbezeichnung "Arkon P 100" im Handel erhältlich.

Das in der Tabelle I genannte α-Methyl-
styrolharz mit einem Erweichungspunkt von 120° C diente
als Verstärkungsharz. Es ist ein Produkt, das im Handel
unter dem Markennamen "Kristallex F 120" erhältlich
ist.

Das in der Tabelle genannte aliphatisch/
naphthenische Oel wies eine Kohlenwasserstoffverteilung
von 2% aromatischen Kohlenwasserstoffen 32 Gew.-% naphthenischen Kohlenwasserstoffen und 66 % paraffinischen
Kohlenwasserstoffen auf, und diese Kohlenwasserstoffverteilung ist in der vorangegangenen Tabelle I als
$C_A = 2$ %, $C_N = 32$ %, $C_P = 66$ % angegeben. Bei den restlichen vier Komponenten der vorangegangenen Tabelle I
handelt es sich um Stabilisatoren, bzw. Lichtschutzmittel.

Zur Herstellung der thermoplastisch verarbeitbaren Elastomerkomponente wurden die in der
Tabelle angeführten Komponenten in einem Knetwerk mit
Z-förmigen, ineinander greifenden Knetarmen, also einem
sogenannten Z-Kneter, bei einer Temperatur von 160° C
miteinander vermischt, und zwar wurde in den Z-Kneter
zuerst das Oel, zusammen mit den Stabilisatoren, eingeführt. Nachdem die Verarbeitungstemperatur von etwa
160° C erreicht war, wurde das SEBS-Copolymerisat zugegeben und es wurde eine Stunde lang homogenisiert.

Dr.IM.-vd
25.11.1985              - 38 -

Anschliessend erfolgte innerhalb von 30 Minuten die Zugabe des cycloaliphatischen Harzes und des α-Methyl-styrolharzes. Es wurde noch zwei weitere Stunden lang bei der angegebenen Temperatur geknetet.

Die so erhaltene fertige Mischung besass bei einer Temperatur von 160° C eine Viskosität von 90'000 mPas. Diese Mischung stellt ein zur Durchführung des erfindungsgemässen Verfahrens geeignetes thermoplastisch verarbeitbares Elastomermaterial dar.

Von dieser Mischung wurde mittels Breitschlitzdüse ein 20 mm breiter, 0,2 mm starker Film extrudiert und nach Erkalten und Konditionierung das Zug/Drehungsverhalten in eine Zugprüfmaschine bei einer Prüfgeschwindigkeit von 500 mm/min untersucht. Die Konditionierung erfolgte durch 24-stündige Lagerung bei 22° C und einer Raumfeuchtigkeit von 55 %.

Bei der Bestimmung des Kraft/Dehnungsverhaltens wurde für den absoluten Wert der Kraft $\sigma$ bei den angegebenen Prozenten der Dehnung $\varepsilon$ die in der folgenden Tabelle angegebenen Werte erhalten:

## Tabelle II

| $\sigma = \dfrac{F}{q}$ (N/mm2) | $\varepsilon$ bei % |
|---|---|
| 0,4 | 50 |
| 0,5 | 100 |
| 0,6 | 150 |

Die Verstreckungsgrenze Vg betrug 200 %. Der Verformungsgrad VF wurde so bestimmt, wie dies weiter vorne

Dr.IM.-vd
25.11.1985

im Abschnitt II im Zusammenhang mit der Bestimmung der Retardation erläutert ist. Der Verformungsgrad betrug 8 % nach 3 Stunden bei einer Temperatur von 20° C und 14 % nach 3 Stunden bei einer Temperatur von 40° C.

Beispiel 2

<u>Herstellung eines thermoplastisch verarbeitbaren Elastomermaterials</u>

Zur Herstellung des Elastomermateriales wurden die in der nachfolgenden Tabelle III angeführten Bestandteile in den dort eingesetzten Mengenverhältnissen verwendet.

Dr.IM.-vd
25.11.1985                — 40 —

Tabelle III

| Bestandteil | Menge in Gew.-% |
|---|---|
| radial aufgebautes SBS-Blockcopolymerisat (Styrol/Butadien-Verhältnis 43/57) | 45 |
| Cycloaliphatisches Harz (Erweichungspunkt ASTM 2398: 100°C) | 15 |
| α-Methylstyrolharz (Erweichungspunkt ASTM 2398: 120°C) | 15 |
| aliphatisch / naphtenisches Oel ($C_A$ = 2% $C_N$ = 32% $C_P$ = 66%) | 23 |
| Alkyliertes, aryliertes-bisphenol-phosphit | 0,5 |
| Zinkdibutyldithiocarbamat | 0,6 |
| 2,2' Methylen- bis [6- (1-methylcyclohexyl)-p-kresol] | 0,5 |
| Pentaerythrityl tetrakis (3,5-di-tert.butyl-4hydroxy-hydro cinnamat ) | 0,2 |
| 2-(2'Hydroxy - 5'methylphenyl) benzotriazol | 0,2 |

Das in der Tabelle III angeführte radial aufgebaute SBS-Blockcopolymerisat besteht aus 43 % Styrol und 57 % Butadien, also es weist ein Styrol/Butadienverhältnis von 43/57 auf. Aufgrund der radialen Struktur dieses Blockcopolymerisates besitzt es eine hohe Zugfestigkeit und gleichzeitig eine geringe Viskosität bei der Verarbeitungstemperatur. Das zugegebene SBS-Blockcopolymerisat ist im Handel unter der Markenbezeichnung "Firestone 840 AA" erhältlich.

Das in der Tabelle III angeführte cyclo-aliphatische Harz, sowie das α-Methylstyrolharz und

das aliphatisch/naphthenische Oel waren die gleichen Produkte, die auch im Beispiel 1 verwendet wurden.

Im Gegensatz zu den gesättigten Block-Blockcopolymeren, die gemäss Beispiel 1 verwendet wurden, neigen die im vorliegenden Beispiel eingesetzten SBS-Blockcopolymeren aufgrund noch vorhandener freier Doppelbindungen zur Polymerisation während der Verarbeitung. Deshalb kommt der Stabilisierung eine besondere Bedeutung zu. Insbesondere das verwendete alkylierte, arylierte Bisphenol-phosphit, das im Handel unter der Markenbezeichnung "Agerite Geltrol" erhältlich ist, und das verwendete p-Kresol-Derivat, das im Handel unter dem Markennamen "Permanox WSP" erhältlich ist, verhindern bei Konzentration von 0,2 - 1 % im ausreichenden Masse die Polymerisation. Eine Polymerisation lässt sich durch eine Viskositätszunahme und eine Hautbildung feststellen.

Die Herstellung der Mischung des thermoplastisch verarbeitbaren Elastomermateriales aus den in der Tabelle III angegebenen Komponenten wurde nach dem in Beispiel 1 beschriebenen Verfahren durchgeführt.

Die so erhaltene Mischung wies bei einer Temperatur von 160° C eine Viskosität von 120 000 mPas auf. Die übrigen Eigenschaften wurden nach dem in Beispiel 1 beschriebenen Verfahren bestimmt. Man erhielt dabei die in der nachfolgenden Tabelle IV angegebenen Werte:

Dr.IM.-vd
25.11.1985

0187270

Tabelle IV

$$\sigma = \frac{F}{q} \ [N/mm2]$$ $\quad\quad\quad\quad\quad\quad\quad\quad \varepsilon \ \%$

| $\sigma = \frac{F}{q}$ [N/mm2] | $\varepsilon$ % |
|---|---|
| 0,3 | 50 |
| 0,4 | 100 |
| 0,5 | 150 |

Die Verstreckungsgrenze Vg lag bei 180 %.

Der Verformungsgrad VF betrug 9 % nach 3 Stunden bei einer Temperatur von 20° C und 18 % nach 3 Stunden bei einer Temperatur von 40° C.

Beispiel 3

Herstellung eines thermoplastisch verarbeitbaren Elastomermateriales

Zur Herstellung des Elastomermateriales wurden die in der folgenden Tabelle V angegebenen Bestandteile in den dort angegebenen Mengenverhältnissen verwendet:

Dr.IM.-vd
25.11.1985

## Tabelle V

| Bestandteile | Menge Gew. % |
|---|---|
| Styrol-Äthylen-Butylen-Styrol Copolymerisat (Styrol/Kautschuk-Verhältnis 29/71) | 36 |
| Cycloaliphatisches Harz (Erweichungspunkt ASTM 2398: 85 ° C) | 10 |
| Aromatisches Harz (Erweichungspunkt ASTM 2398: 158° C) | 17 |
| aliphatisches / naphtenisches Oel ($C_{A,N}$ = 17 %  $C_P$ = 82,5 %) | 36 |
| Zinkdibutyldithiocarbamat | 0,4 |
| Pentaerythrityl-tetrakis (3,5 - di - tert.butyl-4hydroxy hydro cinnamat) | 0,4 |
| 2-(2'-Hydroxy-5'-methylphenyl) benzotriazol | 0,2 |

Bei diesem Beispiel wurde ein SEBS-Block-copolymer verwendet, das bei einer Zugfestigkeit von 32 MN/mm$^2$ und 500 % Bruchdehnung eine niedrigere Verarbeitungsviskosität besass, als das in Beispiel 1 verwendete SEBS-Blockcopolymer. Es bestand aus 21 % Styrol und 71 % Kautschuk. Das Produkt ist im Handel unter der Markenbezeichnung "Kraton G 1652" erhältlich.

Das Mittelblock verträgliche Harz war ein cycloaliphatisches Harz, nämlich ein hydriertes Kohlenwasserstoffharz aus Monomerbestandteilen mit 5 - 9 Kohlenstoffatomen. Es besass einen Erweichungspunkt, bestimmt nach der Methode ASTM 2398 von 85° C und ein Molekulargewicht von 600. Das Produkt ist im Handel unter der Markenbezeichnung "Arkon P 85" er-

Dr.IM.-vd
25.11.1985

hältlich.

Das zur Verstärkung der Endblockdomänen dienende aromatische Harz war ein Kohlenwasserstoffharz mit hohem Erweichungspunkt, nämlich einem Erweichungspunkt, bestimmt nach der Methode von ASTM 2395 von 158° C. Dieses Produkt besass eine Glasübergangstemperatur (tg) von 108° C. Das Produkt ist im Handel unter der Markenbezeichnung "Endex 160" erhältlich.

Das hier eingesetzte aliphatisch/naphthenische Oel bestand hauptsächlich aus Paraffinen, denn es wies einen Paraffingehalt von 82,5 % auf. Damit konnte erreicht werden, dass dieses Oel hauptsächlich nur die Kautschukphase plastifizierte und nur wenig die Polystyrolendsegmente. Das Oel ist im Handel unter der Markenbezeichnung "Shellflex 210" erhältlich.

Die Mischung der in der Tabelle V angeführten Komponenten wurde ähnlich durchgeführt, wie dies in den Beispielen 1 und 2 beschrieben ist. Die Produkte wurden nach dem in Beispiel 1 beschriebenen Verfahren bezüglich ihrer physikalischen Eigenschaften gemessen.

Das hier hergestellte Elastomermaterial besass bei einer Temperatur von 160° C eine Verarbeitungsviskosität von 130'000 mPas.

Bei der Bestimmung des Kraft/Dehnungsverhaltens wurden die in der nachfolgenden Tabelle VI angeführten Werte erhalten:

Dr.IM.-vd
25.11.1985

## Tabelle VI

$$\sigma = \frac{F}{q} \quad (N/mm2) \qquad\qquad \varepsilon \text{ bei } \%$$

| $\sigma$ | $\varepsilon$ |
|---|---|
| 0,4 | 50 |
| 0,5 | 100 |
| 0,6 | 150 |

Die Verstreckungsgrenze wurde bei 200 % beobachtet.

Der Verformungsgrad VF betrug 5 % nach 3 Stunden bei 20° C und 8 % nach 3 Stunden bei 40° C.

## Beispiel 4

### Herstellung einer Hotmeltkomponente

Die nach diesem Beispiel hergestellte Hotmeltkomponente ist zur Coextrusion mit dem thermoplastisch verarbeitbaren Elastomermaterial der Beispiele 1, 2 oder 3 geeignet.

Es wurde ein Hotmelt hergestellt, der ein Haftschmelzklebestoff ist und gute Wärmebeständigkeitseigenschaften aufweist. Diese Eigenschaften sind bevorzugte Eigenschaften der zur Durchführung des erfindungsgemässen Verfahrens eingesetzten Hotmeltklebestoffe.

Zur Herstellung des Hotmeltklebestoffes wurden die in der nachfolgenden Tabelle VII angegebenen Bestandteile in den dort genannten Mengen eingesetzt.

Dr.IM.-vd
25.11.1985

## Tabelle VII

| Bestandteil | Menge in Gew.% |
|---|---|
| Styrol/Isopren/Styrol-Copolymer (Styrol/Isopren-Verhältnis 14/86) | 35 |
| d-Limonen-Harz (Erweichungspunkt 115°C) | 30 |
| Terpen-Phenolharz (Erweichungspunkt 100°C) | 20 |
| Aliphatisch-naphtenisches Oel ($C_{A,N}$ = 23%  $C_P$ = 72%) | 13 |
| Zinkdibutyldithiocarbamat | 1,0 |
| Triphenylphosphit | 0,6 |
| Pentaerythrityl tetrakis (3,5-di-tert.butyl-4-hydroxhydrocinnamat) | 0,2 |
| 2'(2'-Hydroxy-5'methylphenyl) benzotriazol | 0,2 |

Das Styrol/Isopren/Styrol-Copolymere wird in der Folge als SIS-Blockcopolymer abgekürzt. Aus SIS-Blockcopolymeren können durch den Zusatz von Harzen stark klebrige Haftklebestoffe hergestellt werden. Das hier verwendete, in der Tabelle VII angeführte SIS-Blockcopolymere weist ein Styrol/Isopren-Verhältnis von 14 % Styrol zu 86 % Isopren auf. Es ist ein Produkt, das im Handel unter der Markenbezeichnung "Cariflex 1107" erhältlich ist.

Als klebrigmachendes Harz wurde ein Polymerisat auf Basis von d-Limonen verwendet. Das hier eingesetzte Produkt weist eine breite Molekulargewichtsverteilung auf und dadurch ein ausgewogenes Kohäsions-Adhäsionsverhältnis.

Das in Tabelle VII genannte Copolymer-

Dr.IM.-vd
25.11.1985

harz aus Terpen und Phenol wurde der Formulierung zugegeben, um der so hergestellten Hotmeltkomponente eine höhere Kriechbeständigkeit zu verleihen. Diese Harze sind für die vorgesehenen Zwecke gut geeignet. Ein Zusatz von Verstärkungsharzen auf rein aromatischer Basis wirkt hingegen nicht vorteilhaft, weil dadurch die Haftkraft der Formulierung stark vermindert würde. Das hier verwendete Terpen-Phenolharz wies einen Erweichungspunkt von 100° C auf. Dieses Produkt ist im Handel unter dem Markennamen "Dertophene T" erhältlich.

Als Oelkomponente wurde ein aliphatisch naphthenisches Oel verwendet, das einen Gehalt von 23 Gew.-% an aromatischen und naphthenischen Bestandteilen aufwies und 72 Gew.-% an Paraffinbestandteilen enthielt.

Die vier weiteren Komponenten, die in der Tabelle VII angeführt sind, dienen als Stabilisatoren.

Zur Herstellung des Haftschmelzklebestoffes wurde in ein beheiztes Rührwerk zunächst die Oelkomponente, die halbe Menge des d-Limonen-Harzes und die gesamte Menge an Stabilisatoren eingebracht. Es wurde gerührt und erhitzt und nachdem die Verarbeitungstemperatur von 160° C erreicht war, wurde das SIS-Blockcopolymerisat zugesetzt und 1 1/2 Stunden lang weiter homogenisiert. Anschliessend wurde die restliche Menge des d-Limonen-Harzes, dessen Erweichungspunkt 115° C beträgt, und die gesamte Menge des Terpen-Phenolharzes, dessen Erweichungspunkt 100°C beträgt, zugesetzt, und es wurde noch eine weitere

Dr.IM.-vd
25.11.1985

Stunde gerührt. Die so erhaltene Mischung wies bei der Temperatur von 160° C eine Verarbeitungsviskosität von 12000 mPas auf. Der Erweichungspunkt der Formulierung betrug 90° C.

Die Haftfestigkeit und die Kriechbeständigkeit dieses Hotmeltklebestoffes wurde in Kombination mit dem gemäss Beispiel 1 hergestellten thermoplastisch verarbeitbaren Elastikmaterial bestimmt. Zu diesem Zwecke wurde eine Coextrusion des gemäss dem vorliegenden Beispiel hergestellten Hotmeltklebestoffes mit dem Elastikmaterial des Beispiels 1 durchgeführt, und zwar so, dass die Elastikschicht eine Breite von 10 mm und eine Dicke von 0,2 mm aufwies und die Hotmeltschicht in unterschiedlichen Auftragsgewichten aufgebracht wurde. Die Auftragsgewichte der Hotmeltschicht waren 40 g/m$^2$, bzw. 60 g/m$^2$, bzw. 80 g/m$^2$.

Die in der angegebenen Dicke aufgebrachte Schicht des Elastikmaterials wies eine Zugfestigkeit von 1 N bei 100 % Dehnung auf, also eine Zugfestigkeit von 0,5 N/mm$^2$ bei 100 % Dehnung.

Für diesen Test wurde die Coextrusion auf ein mit Silikon imprägniertes Trennpapier durchgeführt. Ein 10 cm langer Streifen wurde nach dem Erkalten von dem Trennpapier gelöst und auf 20 cm gedehnt, sodass eine Dehnung oder Verstreckung auf 100 % durchgeführt wurde. In gedehntem Zustand wurde dieser Streifen so an eine Folie angedrückt, dass die Schicht des Haftklebers mit dieser Folie in Berührung kam.

Als Folie wurde eine Folie aus Polyäthylen geringer Dichte (low density polyethylene foil) verwendet, die als LDPE-Folie abgekürzt wird. Diese Folie wies eine Dicke von 27 µm auf.

Die Haftfestigkeit wurde dann nach dem Verfahren getestet, das weiter vorne unter Punkt IV beschrieben ist, indem man die aufgeklebte Schicht des Elastikmateriales von der Folie in einem Winkel von 180° abzog. Dadurch erhielt man die in der folgenden Tabelle VIII angegebenen Werte:

### Tabelle VIII

| Menge an Hotmeltschicht in g/m2 | Haftfestigkeit in N/10 mm |
|---|---|
| 40 | 3,0 |
| 60 | 3,5 |
| 80 | 4,2 |

### Beispiel 5

### Herstellung einer Hotmeltkomponente

Es wurde eine Hotmeltkomponente hergestellt, die zusammen mit dem thermoplastisch verarbeitbaren Elastomermaterial der Beispiele 1, 2 oder 3 auf die Oberfläche von bahnförmigen Materialien durch Coextrusion oder nacheinander erfolgende Extrusion aufgebracht werden kann.

Die nach dem vorliegenden Beispiel hergestellte Hotmeltschicht ist zur Durchführung des erfindungsgemässen Verfahrens nach derjenigen Variante geeignet, bei der das coextrudierte Produkt in unge-

Dr.IM.-vd
25.11.1985

streckter Form in heissem Zustand direkt mit dem vorgefalteten bahnförmigen Material verklebt wird, wobei während des Aufklebens so weit abgekühlt wird, dass die Elastikkomponente ihre elastischen Eigenschaften noch während des Aufklebens erhält. Dieses Arbeitsverfahren wird im Beispiel 8 unter Bezugnahme auf Fig. 2 der Zeichnung noch näher erläutert.

Bei dieser Ausführungsart des erfindungsgemässen Verfahrens muss die Hotmeltschicht nicht zwingendermassen aus einem Haftschmelzklebstoff bestehen, sondern es kann auch eine Hotmeltschicht eingesetzt werden, die innerhalb einer bestimmten offenen Zeit mit dem bahnförmigen Material verklebt werden muss. Derartige Hotmeltschichten werden als "Hotmeltschicht mit begrenzter offener Zeit" bezeichnet.

Zur Herstellung der Hotmeltschicht mit begrenzter offener Zeit wurden die in der folgenden Tabelle IX angeführten Bestandteile in den dort angegebenen Mengen eingesetzt:

Dr.IM.-vd
25.11.1985

## Tabelle IX

| Bestandteil | Menge in Gew. % |
|---|---|
| Äthylen-Vinylacetat-Copolymer (VA-Gehalt 33%, MI = 45) | 38 |
| Butylbenzylphtalat | 8 |
| Dicyclopentadienharz (Erweichungspunkt 100°C) | 33,5 |
| Pentaerythrytester von durch Hydrierung stabilisiertem Kolophonium (Erweichungspunkt 100°C) | 20 |
| Dilauryl-thiopropionat | 0,2 |
| Pentaerythrityl-tetrakis (3,5-di-tert.butyl-4hydroxy-hydro-cinnamat) | 0,2 |
| 2-(2'-Hydroxy-5'methylphenyl) benzotriazol | 0,1 |

Das in der Tabelle IX genannte Aethylen-Vinylacetat-Copolymer weist einen hohen Gehalt von Vinylacetat von 33 % auf und besitzt somit eine hohe Polarität. Das fragliche Produkt ist im Handel unter der Markenbezeichnung "Elvax 150" erhältlich.

Die Klebrigmachung dieses Aethylen-Vinylacetat-Copolymers wurde durch die Zugabe des Dicyclopentadienharzes und des Esters des durch Hydrierung stabilisierten Kolophoniums erreicht. Das verwendete Dicyclopentadienharz war ein durch Hydrieren stabilisiertes Cyclopentadienharz. Durch die Hydrierung der Kolophoniumkomponente des Kolophoniumesterharzes wurde ebenfalls eine Stabilisierung erreicht.

Das fragliche Harz wies starke polare Eigenschaften auf. Es ist im Handel unter der Markenbezeichnung "Superester A 100" erhältlich. Das verwendete Dicyclopentadienharz ist im Handel unter der Markenbezeichnung "Escorez 5300" erhältlich.

Die beiden Harze wurden so ausgewählt, dass sie beide den gleichen Erweichungspunkt, nämlich einen Erweichungspunkt von 100° C aufwiesen.

Das in der Tabelle IX genannte Butylbenzylphthalat dient als Weichmacher.

Zur Verlängerung der offenen Zeit des Hotmeltklebers mit begrenzter offener Zeit kann der oben angegebenen Formulierung als weitere Komponente ein Phthalsäureester-Adukt zugesetzt werden.

Die drei letzten Komponenten der in Tabelle IX angeführten Formulierung dienen als Stabilisatoren.

Zur Herstellung der Hotmeltkomponente wurde ein beheiztes Rührwerk verwendet. Zunächst wurde der Weichmacher zusammen mit den Stabilisatoren in das Rührwerk eingebracht, und man erhitzte unter Rühren auf die Verarbeitungstemperatur von 160° C. Anschliessend wurde das Aethylen-Vinylacetat-Copolymer zugegeben und es wurde bei der angegebenen Temperatur eine Stunde lang weitergerührt, bis sich dieses Copolymer vollständig gelöst hatte. Anschliessend wurde das Dicyclopentadienharz, zusammen mit dem Kolophoniumesterharz, zugesetzt und man rührte nach der Zugabe dieser beiden Harze bei der angegebenen Temperatur noch eine Stunde weiter.

Nach dem in Beispiel 4 beschriebenen Verfahren wurde die Hotmeltkomponente des vorliegenden Beispiels zusammen mit der Elastikkomponente des Beispiels 1 coextrudiert. Im vorliegenden Fall wurde jedoch das Produkt nicht auf ein Trennpapier extrudiert, sondern das Produkt wurde direkt nach der Coextrusion der LDPE-Folie zukaschiert. Es wurde wieder eine LDPE-Folien einer Dicke von 27 µm verwendet. Bei diesem Test wurde die Folie nicht vorgefaltet, sodass kein elastisches Produkt entstand. Da jedoch nur die Haftfestigkeit der Hotmeltklebeschicht getestet werden sollte, störte dies nicht.

Die Testung der Haftfestigkeit wurde in der gleichen Weise durchgeführt, wie in Beispiel 4 beschrieben , und zwar durch Abschälversuche unter einem Winkel von 180°, unter Verwendung einer Zugprüfmaschine. Man erhielt dabei die in der nachfolgenden Tabelle X angegebenen Werte für die Haftfestigkeit:

### Tabelle X

| Menge an Hotmeltschicht in g/m2 | Haftfestigkeit in N/10 mm |
|---|---|
| 40 | 1,0 |
| 60 | 1,4 |
| 80 | 1,7 |

### Beispiel 6

### Verfahren zur Befestigung eines elastischen Bandes auf einem vorgefalteten, bahnförmigen Material

Das hier beschriebene Verfahren wurde mit jeder der in den Beispielen 1, 2 und 3 beschriebenen Elastikmaterialien in Kombination mit der in Beispiel 4 beschriebenen Hotmeltkomponente durchgeführt. Das Verfahren wird anhand der Fig. 3 näher erläutert.

Es wurde die in Fig. 3 links dargestellte Extrusionsdüse verwendet, mit Hilfe der das thermoplastisch verarbeitbare Material (2) und das Hotmeltmaterial (1) so extrudiert wurde, dass auf dem Förderband (13) die Schicht des thermoplastischen Materiales (2) aufliegt und sich darüber die Schicht des Hotmeltmateriales (1) befindet.

Die Coextrusionsdüse (6) ist an ein zirkulierendes Hotmeltauftragungsgerät angeschlossen, das ein gleichmässiges Fliessverhalten im ganzen System gewährleistet. Damit wird ein fadenfreies intermittierendes Auftragen des coextrudierten Produktes (7) auf das Förderband (13) ermöglicht.

Die zur Coextrusion verwendete Düse (6) ist so beschaffen, dass ein laminarer Strömungsfluss entsteht und so die Schicht des Hotmeltmateriales (1) sauber getrennt über der Schicht des Elastomermateriales (2) extrudiert wird. In der Extrusionsdüse (6) müssen turbulente Strömungsverhältnisse vermieden werden, weil dies zu einer teilweisen oder vollständigen Vermischung des Hotmeltmateriales (1) mit dem Elastomermaterial (2) in der Düse, bzw. am Düsenaustritt führen würde.

In Fig. 3 ist bei 6a eine andere Ausführungsart der Extrusionsdüse veranschaulicht, die eine Extrusion von insgesamt drei Schichten ermöglicht, und zwar einer Schicht des Elastomermateriales (2),

Dr.IM.-vd
25.11.1985

die von der Schicht des Hotmeltmateriales (1) durch eine Schicht eines weiteren Materiales (3) getrennt ist. Diese Schicht (3) des weiteren Materiales kann entweder aus einer Schutzschicht bestehen, die einen direkten Kontakt zwischen der Schicht des Elastomer- materiales (2) und der Schicht des Hotmeltmateriales (1) verhindert, oder sie kann auch eine Schicht eines weiteren thermoplastisch verarbeitbaren Elastomermateriales sein, das andere Elastikeigenschaften besitzt, als das Elastomermaterial (2).

Das coextrudierte Produkt wird auf das Förderband oder Transferband (13) extrudiert. Dieses Transferband ist abweisend ausgerüstet, damit nach dem Abkühlen des coextrudierten Produktes (7) eine leichte Loslösung des aufliegenden Elastikmateriales von dem Transferband gewährleistet ist.

Das coextrudierte Produkt wird auf dem Transferband gekühlt, damit gewährleistet ist, dass die Elastikkomponente (2) bereits ihre elastischen Eigenschaften erreicht hat, sobald das coextrudierte Produkt auf das bahnförmige Material (4) aufgeklebt wird. Das bahnförmige Material (4) wird von einer Rolle (12) kontinuierlich abgewickelt und mit Hilfe der Walze (10) vorgefaltet und in vorgefaltetem Zustand an das Förderband (13), unter Mitwirkung der Walze (14) angepresst. Sobald ein coextrudiertes Produkt (7) diese Stelle passiert, wird der Hotmeltkleber (1) mit den Spitzen des vorgefalteten bahnförmigen Materiales (4) verklebt. Sofort anschliessend passiert die Materialbahn die Andruckvorrichtung (5). Diese Andruckvorrichtung (5) ist eine Vakuumanpress-

Dr.IM.-vd
25.11.1985

Station, die den nötigen Druck ausübt, damit das zunächst nur an den Spitzen angeklebte coextrudierte Produkt (7) vollflächig mit den entsprechenden Stellen des bahnförmigen Materiales (4) verklebt wird. An denjenigen Stellen des bahnförmigen Materials, an denen kein extrudiertes Produkt (7) aufgeklebt wurde, löst sich die Vorfaltung hinter der Vakuumpress-Station wieder auf. An den Stellen, wo jedoch das coextrudierte Produkt (7) auf die Materialbahn aufgeklebt wurde, bleibt die Vorfaltung erhalten, und die fragliche Stelle ist dann durch das aufgeklebte Elastikband elastisch dehnbar.

Beispiel 7

Verfahren zur Befestigung eines vorgestreckten elastischen Bandes auf einem nicht vorgefalteten, bahnförmigen Material

Das hier beschriebene Arbeitsverfahren wird anhand der Fig. 4 näher erläutert.

Bei dieser Variante wird in ähnlicher Weise wie bei dem in Beispiel 6 beschriebenen Verfahren zunächst durch Coextrusion ein coextrudiertes Produkt (7) hergestellt, und zwar so auf ein Förderband (13) extrudiert, dass wiederum die Schicht des Elastomermateriales (2) auf dem Förderband aufliegt, während sich die Schicht des Hotmeltmateriales (1) auf dieser Schicht des Elastomermateriales befindet.

Das coextrudierte Produkt wird auf dem

Dr.IM.-vd
25.11.1985

Förderband (13) gekühlt, bis das Elastomermaterial seine elastischen Eigenschaften erreicht hat. Die Geschwindigkeit, mit der das zur Kühlung dienende Transportband (13) sich fortbewegt, ist geringer als die Geschwindigkeit, mit der sich das Walzenpaar (17, 17) bewegt. Durch diese Differenzgeschwindigkeit wird der gewünschte Verstreckungsgrad des coextrudierten Produktes erreicht.

Ueber die Walze (12) wird das bahnförmige Material (4) zu der Walze (15) im nicht gerafften Zustand zugeführt und die Verklebung des gestreckten, coextrudierten Produktes mit der Materialbahn (4) erfolgt durch den Druck der Walzen (15) und (16). Anschliessend wird dann entspannt, wobei durch die Rückstellkräfte des aufgeklebten Elastikbandes das bahnförmige Material (4) an denjenigen Stellen gerafft wird, wo das Elastikband in gestrecktem Zustand aufgeklebt wurde. Bei dem hier beschriebenen Verfahren wurde als bahnförmiges Material (4) eine Polyäthylenfolie geringer Dichte verwendet, die entsprechend der englischsprachigen Benennung, die "low density polyethylene foil" lautet, mit LDPE-Folie abgekürzt wird.

Bei dem im vorliegenden Beispiel beschriebenen Verfahren und auch bei dem in Beispiel 6 beschriebenen Verfahren, ist es wesentlich, dass eine Selbstklebeeigenschaften aufweisende Hotmeltschicht eingesetzt wird, denn in beiden Fällen muss dort, wo das coextrudierte Produkt (7) im erkalteten Zustand, wo es bereits seine Elastikeigenschaften erreicht hat, noch gute Klebeeigenschaften aufweisen, damit eine Haftung des coextrudierten Produktes auf dem
bahnförmigen Material (4) gewährleistet ist.

Beispiel 8

Verfahren zum Befestigen eines elastischen Bandes auf einem vorgefalteten, bahnförmigen Material

Das hier beschriebene Verfahren hat eine
gewisse Aehnlichkeit mit dem in Beispei 6 beschriebenen Verfahren. Hier wird jedoch die Coextrusion
so durchgeführt, dass die Hotmeltschicht mit der
darauf befindlichen Schicht des Elastomermateriales
direkt auf das vorgefaltete, bahnförmige Material extrudiert wird und in heissem Zustand mit dem vorgefalteten, bahnförmigen Material verklebt wird. Es
wird also im Unterschied zu Beispiel 6 keine Kühlung
des coextrudierten Materiales (7) vor dessen Uebertragung auf das vorgefaltete, bahnförmige Material
vorgenommen.

Bei diesem Verfahren wurde jeweils eine
Elastikkomponente des Beispiels 1, des Beispiels 2,
und des Beispiels 3, mit einer Hotmeltschicht des Beispiels 4 coextrudiert und ebenfalls eine Coextrusion
einer Elastikkomponente des Beispiels 1, des Beispiels
2 und des Beispiels 3 mit einer Hotmeltkomponente des
Beispiels 5 vorgenommen.

Das hier beschriebene Verfahren wird in
Fig. 2 schematisch veranschaulicht.

Das bahnförmige Material (4) wird von
der Rolle (12) der Walze (10) zugeführt und mit Hilfe

Dr.IM.-vd
25.11.1985                    - 59 -

der Walze (10) gefaltet. Es muss beachtet werden, dass die Faltung so eng gehalten wird, dass ein Eindringen des heissen, flüssigen Coextrudates aus der Hotmeltschicht (1) und der Elastikschicht (2) in die Faltung verhindert wird. Ein solches Eindringen würde nämlich zu einem ungleichmässigen Auftragen und zu uneinheitlichen Elastikeigenschaften des aufgebrachten Elastikbandes führen. Sobald das flüssige Coextrudat so auf das vorgefaltete, bahnförmige Material aufgetragen wurde, dass die Hotmeltschicht auf dem vorgefalteten Substrat aufgeklebt wird und die noch heisse Schicht des Elastikmateriales sich darüber befindet, muss sofort gekühlt werden, damit das Elastikmaterial rasch so weit abgekühlt wird, dass es seine elastischen Eigenschaften erhält. Dementsprechend ist die Kaschierwalze (11) ein gekühlter Zylinder. Sobald die mit der aufgeklebten Schicht des Elastikmateriales versehene Materialbahn (4) den Kühlzylinder (11) verlässt, muss das Elastikmaterial bereits so stark abgekühlt sein, dass es seine Elastikeigenschaften erreicht hat und nicht durch sogenannten inneren Fluss gedehnt wird, sodass die Vorfaltung verloren geht.

Auch in diesem Fall kann die Schicht aus dem Coextrudat des Hotmeltklebers (1) und des Elastikmaterials (2) kontinuierlich oder diskontinuierlich auf das bahnförmige Material (4) aufgebracht werden. Bei einer diskontinuierlichen Aufbringung bleibt die Vorfaltung des Substrates nach dem Verlassen der gekühlten Kaschierwalze (11) nur an den Stellen erhalten, wo das coextrudierte Produkt aufgebracht wurde. Diese Stellen besitzen dann die gewünschten elastischen Eigenschaften. An denjenigen Teilen des bahnförmigen Materiales, an denen kein Coextrudat aufgebracht wurde, löst sich die Vorfaltung auf, nachdem die Materialbahn die gekühlte Kaschierwalze verlassen hat.

Es zeigte sich, dass bei der in diesem Beispiel beschriebenen Verfahrensweise die Hotmeltschicht gemäss Beispiel 5 zu besseren Ergebnissen führt als die Hotmeltschicht gemäss Beispiel 4. Die Hotmeltschicht gemäss Beispiel 5 weist, wie bereits erwähnt wurde, eine begrenzte offene Zeit auf. Sie besitzt eine kürzere Abbindezeit und dementsprechend entwickelt sich die innere Festigkeit der Hotmeltschicht (3) während des Kaschierens unter Abkühlung mit der gekühlten Kaschierwalze (11).

Beispiel 9

Jedes der in den Beispielen 1, 2 und 3 beschriebenen Elastikmaterialien wurde in Kombination mit der in Beispiel 4 beschriebenen Hotmeltkomponente auf das bahnförmige Material in ähnlicher Weise aufgebracht, wie dies in Beispiel 6 beschrieben ist. Auch hier wurde mit einer Vorrichtung gearbeitet, die nach dem gleichen Prinzip arbeitet, wie die in Fig. 3 dargestellte Vorrichtung. Es wurden jedoch jetzt statt der Coextrusionsdüse 6 zwei unterschiedliche Extrusionsdüsen eingesetzt, durch die auf das Förderband (13) zunächst die Schicht des thermoplastischen Materiales (2) und auf dieser dann die Schicht des Hotmeltmateriales (1) extrudiert wurde. Es wurde also durch nacheinander erfolgende Extrusion auf dem Förderband ein Produkt erzeugt, das dem durch Coextrusion hergestellten Produkt (7) der Fig. 3 entspricht.

Unter Verwendung von drei aufeinander folgenden getrennten Extrusionsdüsen anstelle der in Fig. 3 rechts unter (6a) dargestellten Düse, wurde auf dem Förderband (13) durch nacheinander erfolgende Extrusion ein aus insgesamt drei Schichten aufgebautes Produkt hergestellt, bei der die Schicht des Elastomermateriales (2) von der Schicht des Hotmeltmateriales (3) durch eine Schicht eines weiteren Materiales (3) getrennt ist. Dieses Material (3) kann wieder ein Material einer Schutzschicht sein oder die Schicht kann aus einem weiteren thermoplastisch verarbeitbaren Elastomermaterial bestehen, das andere Elastikeigenschaften besitzt, als das Elastomermaterial (2).

Das aus zwei oder drei Schichte bestehende Produkt (7) wird auf dem Transferband (13) gekühlt und in der gleichen Weise auf das vorgefaltete bahnförmige Material (4) aufgebracht, wie dies in Beispiel 6 beschrieben ist.

Beispiel 10

In diesem Beispiel wurden die gleichen Materialien verwendet wie in Beispiel 7 und es wurde im Prinzip in der gleichen Weise gearbeitet, wie dies in Beispiel 7 erläutert ist. In diesem Falle wurde jedoch das aus der Schicht des Elastomermateriales

Dr.IM.-vd
25.11.1985

(2) und der Hotmeltschicht (1) aufgebaute Produkt nicht durch Coextrusion sondern durch aufeinander folgende Extrusion auf dem Förderband (13) erzeugt. Auch in diesem Falle erfolgte die Extrusion so, dass die Schicht des Elastomermateriales (2) auf dem Förderband auflag, während sich die Schicht des Hotmeltmateriales (1) auf dieser Schicht des Elastomermateriales befand.

Das aus den beiden Schichten aufgebaute Produkt wurde dann auf dem Förderband (13) gekühlt, gestreckt und im verstreckten Zustand auf das bahnförmige Material (4) aufgebracht. Die Kühlung, Verstreckung und Uebertragung erfolgte in der gleichen Weise, wie dies in Beispiel 7 beschrieben ist.

Beispiel 11

Es wurde unter Verwendung der in Fig. 3 schematisch dargestellten Vorrichtung im wesentlichen in der gleichen Weise gearbeitet, wie in Beispiel 8. In diesem Falle wurde auf das vorgefaltete bahnförmige Material durch aufeinander folgende Extrusion zunächst die Hotmeltschicht (1) und über dieser die Schicht des elastischen Materiales (2) aufgebracht. Die in Fig. 3 dargestellte Düse zur Coextrusion (6) wurde also durch zwei getrennte Extrusionsdüsen ersetzt.

Ansonsten wurden die in Beispiel 8 beschriebenen Materialien verwendet und auch analoge Arbeitsschritte durchgeführt.

Dr.IM.-vd
25.11.1985

Patentansprüche

1. Verfahren zur Befestigung eines elastischen Bandes oder Filmes auf der Oberfläche oder Teilbereichen der Oberfläche von bahnförmigen Materialien, dadurch gekennzeichnet, dass man durch Coextrusion oder nacheinander erfolgende Extrusion ein Produkt herstellt, das aus mindestens einer Hotmeltschicht und mindestens einer Schicht eines thermoplastisch verarbeitbaren Elastomermateriales aufgebaut ist und dieses Material auf das bahnförmige Material kontinuierlich oder diskontinuierlich so in Form eines Bandes oder Filmes aufträgt, dass die Hotmeltschicht des Produktes mit dem bahnförmigen Material verklebt wird.

2. Verfahren nach Anspruch 1, dadurch gekennzeichnet, dass man durch Coextrusion oder nacheinander erfolgende Extrusion ein Produkt herstellt, bei dem sich zwischen der Hotmeltschicht und der Schicht des thermoplastisch verarbeitbaren Elastomermateriales eine Schutzschicht befindet, die einen direkten Kontakt der Hotmeltschicht mit der Schicht des elastischen Materiales verhindert oder ein Produkt herstellt, das zwei oder mehr Schichten des thermoplastisch verarbeitbaren Elastomermateriales aufweist, wobei die unterschiedlichen Schichten des Elastomermateriales unterschiedliche Elastizitäten besitzen und wobei gegebenenfalls ausserdem noch eine Schutzschicht zwischen der Hotmeltschicht und der ihr am nächsten liegenden Schicht des thermoplastisch verarbeitbaren Elastomermateriales vorhanden ist.

3. Verfahren nach Anspruch 1 oder 2, dadurch gekennzeichnet, dass man das durch Coextrusion oder nacheinander erfolgende Extrusion gewonnene Produkt in ungestrecktem Zustand auf das vorgefaltete bahnförmige Material aufträgt.

4. Verfahren nach Anspruch 3, dadurch gekennzeichnet, dass man entweder

a) direkt in heissem Zustand die Hotmeltschicht und über dieser die Schicht des thermoplastisch verarbeitbaren Elastomermaterials durch Coextrusion oder aufeinander folgende Extrusion auf dem vorgefalteten bahnförmigen Material aufklebt und während des Aufklebens kühlt, damit die Schicht des Elastomermaterials ihre elastischen Eigenschaften noch während des Aufklebens erhält oder dass man

b) in heissem Zustand die Schicht des Elastomermateriales und über dieser die Hotmeltschicht durch Coextrusion oder nacheinander folgende Extrusion auf eine Uebertragungsbahn oder einen Uebertragungszylinder so aufträgt, dass das Elastomermaterial mit der Oberfläche des Uebertragungsbandes oder Uebertragungszylinders in Berührung kommt, das extrudierte Produkt auf dem Uebertragungsband oder Uebertragungszylinder so weit abkühlt, dass das Elastomermaterial seine elastischen Eigenschaften erreicht und dann mit der Hotmeltschicht auf das vorgefaltete bahnförmige Material aufklebt, wobei die Hotmeltschicht vorzugsweise aus einem Selbstklebematerial besteht.

5. Verfahren nach Anspruch 1 oder 2, dadurch gekennzeichnet, dass man das durch Coextrusion oder aufeinander folgende Extrusion gewonnene Produkt, das aus der Schicht des Elastomermateriales und der Hotmeltschicht aufgebaut ist, abkühlt, streckt und in gestrecktem Zustand auf das nicht vorgefaltete bahnförmige Material aufklebt, nach dem Aufkleben die Einwirkung der streckenden Kräfte unterbricht, sodass durch die Rückstellkräfte der gestreckten Schicht des Elastomermaterials die Faltung des bahnförmigen Materials erfolgt.

6. Verfahren nach Anspruch 5, dadurch gekennzeichnet, dass man das Material auf einen Kühlzylinder oder ein Kühlband durch Coextrusion oder aufeinander folgende Extrusion so aufbringt, dass die Schicht des Elastomermateriales mit dem Kühlband oder Kühlzylinder in Berührung kommt, und dass man als Hotmeltschicht eine Selbstklebeschicht verwendet, wobei gegebenenfalls diese Hotmeltschicht diskontinuierlich extrudiert wird, sodass das auf den Kühlzylinder, beziehungsweise das Kühlband durch Coextrusion oder nacheinander folgende Extrusion erhaltene Produkt Abschnitte aufweist, die frei von der Hotmeltschicht sind.

7. Verfahren nach einem der Ansprüche 1 bis 6, dadurch gekennzeichnet, dass man als bahnförmiges Material ein folienförmiges Kunststoffmaterial, ein vliesförmiges Material oder ein nicht gewobenes, gewobenes oder gewirktes Textilmaterial verwendet.

Dr.IM.-vd
25.11.1985

8. Verfahren nach einem der Ansprüche 1 bis 7, dadurch gekennzeichnet, dass die Schicht des thermoplastisch verarbeitbaren Elastomermateriales des durch Coextrusion oder nacheinander erfolgende Extrusion gewonnenen Produktes

20 - 70 Gew.-%, bezogen auf das Gesamtgewicht der Schicht des Elastomermateriales eines thermoplastischen Kautschuckes enthält, der ein Dreiblockcopolymer des Types A-B-A ist, wobei die Komponente A Polystyrolsegmente enthält oder daraus aufgebaut ist und die Komponente B den kautschukartigen Mittelblock darstellt, der vorzugsweise aus Isopren- und/oder Butadien-Monomerbestandteilen oder Copolymerisaten mit anderen äthylenisch ungesättigten Monomeren aufgebaut ist oder ein mindestens teilweise hydriertes Copolymerisat aus mindestens einem Diolefin und mindestens einem Monoolefin ist und

10 - 40 Gew.-%, bezogen auf das Gesamtgewicht der Schicht des Elastomermateriales eines aromatischen Kohlenwasserstoffharzes enthält, das vorzugsweise ein Styrolharz mit Glasübergangstemperaturen und Erweichungspunkten von über 100° C ist.

9. Verfahren nach Anspruch 8, dadurch gekennzeichnet, dass die Schicht des thermoplastisch verarbeitbaren Elastomermateriales ausserdem mindestens einen der folgenden weiteren Bestandteile enthält:

a) 0-10 Gew.-%, bezogen auf das Gesamtgewicht der Schicht des Elastomermateriales eines Elastomers, vorzugsweise einen Butylkautschuk,

Dr.IM.-vd
25.11.1985

b) 0-30 Gew.-%, bezogen auf das Gesamtgewicht der Schicht des Elastomermateriales eines aliphatischen Harzes, das eine gute Verträglichkeit mit der Kautschukkomponente B des thermoplastischen Kautschuks aufweist, jedoch eine geringe klebrigmachende Wirkung besitzt und das vorzugsweise ein Kohlenwasserstoffharz mit einem Erweichungspunkt von 10° C bis 100°C ist,

c) 0-35 Gew.-%, bezogen auf das Gesamtgewicht des Elastomermateriales eines Weichmachers, der vorzugsweise ein aliphatisches und/oder napththenisches Oel, Polyisobutylene, ein Phthalsäureester-produkt, ein depolymerisierter Kautschuk oder Mischun-gen aus zwei oder mehr derartiger Weichmacher ist,

d) 0-30 Gew.-%, bezogen auf das Gesamtgewicht der Schicht des Elastomermateriales eines Füllstoffes, der vorzugsweise ein Calciumcarbonat enthaltender oder aus Calciumcarbonat bestehender Füllstoff ist oder

e) 0,2 - 2 Gew.-%, bezogen auf das Gesamtgewicht der Schicht des Elastomermateriales, mindestens eines Stabilisators.

10. Verfahren nach einem der Ansprüche 1 bis 9, dadurch gekennzeichnet, dass man das elastische Band oder den elastischen Film auf dem bahnförmigen Material kontinuierlich oder intermittierend befestigt, und zwar entweder in Maschinenlaufrichtung oder quer zur Maschinenlaufrichtung oder in Kontur-form.

Dr.IM.-vd
25.11.1985

11. Verwendung des nach dem Verfahren gemäss Anspruch 1 hergestellten bahnförmigen Materials, bei dem auf der Oberfläche oder Teilbereichen der Oberfläche das elastische Band oder der elastische Film befestigt ist, für die Herstellung von Fertigprodukten oder Zwischenprodukten, die eine elastisch geraffte Oberfläche oder elastisch geraffte Oberflächenbereiche aufweisen, dadurch gekennzeichnet, dass man das bahnförmige Material zu entsprechenden Endprodukten oder Zwischenprodukten zerschneidet.

12. Verwendung gemäss Anspruch 11, dadurch gekennzeichnet, dass man das bahnförmige Material zur Herstellung von flüssigkeitsabsorbierenden Produkten, beispielsweise Windeln, Produkten für die Monatshygiene, Unterlagen oder Abdecktücher für Operationssäle verwendet oder dass man es zur Herstellung von mit elastischen Abschnitten versehenen Kleidungsstücken, Wäschestücken oder Bett- und Tischwäsche verwendet.

## Fig.1

A
B
C

D
E
F

## Fig. 2

*Fig. 3*

*Fig. 4*

**Europäisches
Patentamt**

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| D,A | EP-A-0 115 286 (BOUSSAC SAINT FRERES)<br>* Seite 7, Zeilen 31-33; Seite 10, Zeilen 26-31 *<br><br>----- | 1 | A 41 B 13/02F<br>B 32 B 25/08 |
| | | | **RECHERCHIERTE SACHGEBIETE (Int Cl 4)**<br><br>A 41 B<br>B 32 B |

Der vorliegende Recherchenbericht wurde für alle Patentansprüche erstellt

| Recherchenort<br>DEN HAAG | Abschlußdatum der Recherche<br>21-03-1986 | Prüfer<br>VAN THIELEN J.B. |
|---|---|---|

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82